# FASCICULE DE BREVET EUROPEEN

(11) **EP 1 050 534 B1**
(45) Date de publication et mention de la délivrance du brevet: **05.12.2001**
(21) Numéro de dépôt: 00401197.9
(22) Date de dépôt: 02.05.2000
(51) Int. Cl.: C07D 401/12, C07D 209/52, C07D 221/04, A61K 31/403, A61K 31/435, C07D 401/14

(54) **Nouveaux dérivés de 2,3-methano-aminoacides, leur procédé de préparation et les compositions pharmaceutiques qui les contiennent**
2,3-Methano-Aminosäure-Derivate, Verfahren zu ihrer Herstellung und diese enthaltende pharmazeutische Zusammensetzungen
2,3-methano-amino acid derivatives, process for their preparation and pharmaceutical compositions containing them

(30) Priorité: 03.05.1999 FR 9905601
(43) Date de publication de la demande: 08.11.2000
(73) Titulaire: LES LABORATOIRES SERVIER, 92200 Neuilly sur Seine (FR)
(72) Inventeur: De Nanteuil, Guillaume, 92150 Suresnes (FR); Gloanec, Philippe, 78380 Bougival (FR); Verbeuren, Tony, 78540 Vernouillet (FR); Rupin, Alain, 37510 Savonnières (FR)

(56) Documents cités:
- EP-A- 0 615 978
- WO-A-95/23609

## Description

La présente invention concerne des nouveaux dérivés de 2,3-méthano-aminoacides, leur procédé de préparation, les compositions pharmaceutiques qui les contiennent ainsi que leur utilisation en tant qu'inhibiteurs de protéases à sérine apparentées à la trypsine.

L'une de ces protéases à sérine, la thrombine, est l'enzyme clé de la coagulation et joue un rôle central dans la pathologie des thromboses veineuses et artérielles, en raison notamment de son fort pouvoir d'auto-amplification de la cascade de la coagulation (F. Toti et coll., Sang, Thrombose, Vaisseaux 1992, 4, 483-494 et T.M. Reilly et coll., Blood Coagulation and Fibrinolysis 1992, 3, 513-517).

L'inhibition directe et spécifique de la thrombine est plus efficace et présente moins de risques d'hémorragie que le traitement par l'héparine. Il existe actuellement des inhibiteurs directs de thrombine, mais ces substances peptidiques présentent l'inconvénient de ne pas être actives par voie orale.

Des dérivés peptidomimétiques, présentant une activité anti-thrombotique orale, ont déjà été décrits dans la littérature. C'est le cas notamment des dérivés de l'acide boronique décrits dans les brevets EP 293 881, EP 471 651, EP 615 978 et EP 792 883 et des dérivés décrits dans les brevets WO 94 29336 et WO 95 23609.

Il était donc particulièrement intéressant de synthétiser de nouveaux inhibiteurs de protéases à sérine afin d'augmenter la puissance et la sélectivité des composés déjà décrits dans la littérature.

De plus, ces nouveaux composés augmentent différents temps de coagulation et sont actifs par voie orale.

Plus spécifiquement, la présente invention concerne les composés de formule (I): 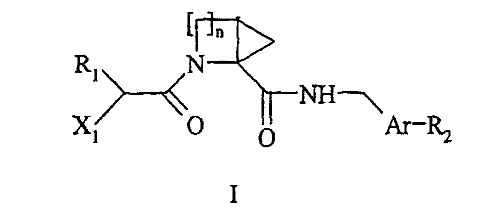 dans laquelle :
◆ n représente 2 ou 3,
◆ R₁ représente un groupement cycloalkyle (C₃-C₈), un groupement phényle éventuellement substitué ou un groupement alkyle (C₁-C₆) linéaire ou ramifié éventuellement substitué par un ou plusieurs groupements, identiques ou différents, choisis parmi halogène, cycloalkyle (C₃-C₈) et phényle éventuellement substitué,
◆ R₂ représente
   - un groupement amino,
   - un groupement amidino substitué éventuellement par un ou plusieurs groupements identiques ou différents, choisis parmi alkyle (C₁-C₆) linéaire ou ramifié, et hydroxy,
   - un groupement guanidino substitué éventuellement par un groupement alkyle (C₁-C₆) linéaire ou ramifié,
   - ou un groupement isothiouréïdo substitué éventuellement par un groupement alkyle (C₁-C₆) linéaire ou ramifié,
◆ Ar représente un groupement aryle ou un groupement hétéroaryle azoté monocyclique,
◆ X₁ représente un groupement hydroxy, un groupement amino ou un groupement -NHR₃,
   - R₃ représente un groupement propargyle, un groupement iminométhyle, un groupement alkylsulfonyle (C₁-C₆) linéaire ou ramifié, un groupement arylalkylsulfonyle (C₁-C₆) linéaire ou ramifié, un groupement -CONR'₃R"₃ ou un groupement alkyle (C₁-C₆) linéaire ou ramifié éventuellement substitué par :
      - un groupement -CO₂R'₃,
      - un groupement -CONR'₃R"₃,
      - un groupement hétérocyclique,
      - un groupement aminosulfonyle,
      - un groupement aryle,
      - ou un groupement hétéroaryle,
         * R'₃ et R"₃, identiques ou différents, représentent chacun un atome d'hydrogène, ou un groupement alkylsulfonyle (C₁-C₆) linéaire ou ramifié, un groupement aryle, un groupement alkyle (C₁-C₆) linéaire ou ramifié (éventuellement substitué par un groupement carboxy, alkoxycarbonyle (C₁-C₆) linéaire ou ramifié ou carbamoyle), ou forment avec l'atome d'azote qui les portent un groupement hétérocyclique,
leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, etc.

Parmi les bases pharmaceutiquement acceptables, on peut citer à titre non limitatif l'hydroxyde de sodium, l'hydroxyde de potassium, la triéthylamine, la tertbutylamine, etc.

Par groupement phényle éventuellement substitué, on entend substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié et amino (substitué éventuellement par un ou plusieurs groupements alkyle (C₁-C₆) linéaire ou ramifié).

Par groupement hétérocyclique, on entend un groupement mono- ou bicyclique, saturé ou insaturé, de 5 à 12 chaînons contenant un, deux ou trois hétéroatomes choisis parmi oxygène, azote ou soufre, étant entendu que l'hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, oxo, hydroxy, trihalogénoalkyle (C₁-C₆) linéaire ou ramifié et amino (substitué éventuellement par un ou plusieurs alkyle (C₁-C₆) linéaire ou ramifié). Les groupements hétérocycliques préférés sont les groupements morpholinyle, pipérazinyle, pipéridyle, dihydro-triazolyle ou imidazolinyle.

Par groupement aryle, on entend phényle, biphénylyle, naphtyle, chacun de ces groupements étant éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs groupements alkyle (C₁-C₆) linéaire ou ramifié) et carboxy.

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié). Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, pyrimidinyle, pyrazinyle, pyridazinyle, pyrazolyle, quinolyle. Les groupements hétéroaryle préférés sont les groupements pyridyle éventuellement substitué ou quinolyle éventuellement substitué.

La configuration relative préférée du groupement 2,3-méthano-aminoacide des composés de formule (I) est cis ((2*R*,3*S*) ou (2*S*,3*R*)). La configuration absolue préférée du groupement 2,3-méthano-aminoacide des composés de formule (I) est (2*S*,3*R*).

Les groupements R₁ préférés de la formule (I) sont les groupements cycloalkyle ou alkyle éventuellement substitué.

Les groupements R₂ préférés de la formule (I) sont les groupements amino ou amidino.

Le terme aryle affecté au groupement Ar tel que défini dans la formule (I) est préférentiellement le groupement phényle éventuellement substitué.

Le terme hétéroaryle azoté monocyclique affecté au groupement Ar tel que défini dans la formule (I) est préférentiellement le groupement pyridyle éventuellement substitué.

Les groupements X₁ préférés de la formule (I) sont les groupements amino ou -NHR₃ dans lequel R₃ représente un groupement arylalkylsulfonyle (C₁-C₆) linéaire ou ramifié ou alkyle (C₁-C₆) linéaire ou ramifié éventuellement substitué.

L'invention s'étend également au procédé de préparation des composés de formule (I) caractérisé en ce que l'on met en réaction un composé de formule (II) : 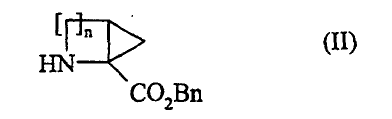 dans laquelle n a la même signification que dans la formule (I), et Bn représente le groupement benzyle,
avec un composé de formule (III): 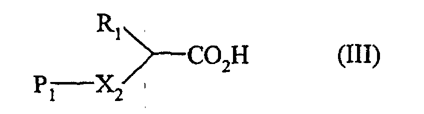 dans laquelle R₁ a la même signification que dans la formule (I), X₂ représente un atome d'oxygène ou -NH-, et P₁ représente un groupement protecteur de la fonction hydroxy ou amino,
en présence d'un agent de couplage peptidique,
pour conduire au composé de formule (IV) : 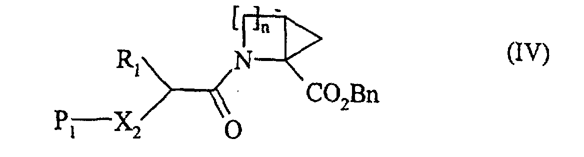 dans laquelle n, R₁, X₂, Bn et P₁ ont la même signification que précédemment,
composé de formule (IV) que l'on transforme ensuite en composé de formule (V) : 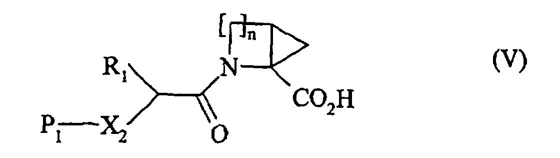 dans laquelle n, R₁, X₂ et P₁ ont la même signification que précédemment, par hydrogénation catalytique ou par saponification,
composé de formule (V) que l'on met ensuite en réaction avec un composé de formule (VI) : 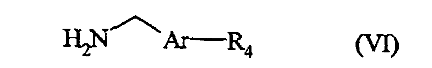 dans laquelle Ar a la même signification que dans la formule (I), et R₄ représente R₂ ou R₂-P₂, R₂ ayant la même signification que dans la formule (I), et P₂ représentant un groupement protecteur de la fonction amino,
en présence d'un agent de couplage peptidique,
pour conduire au composé de formule (VII) : 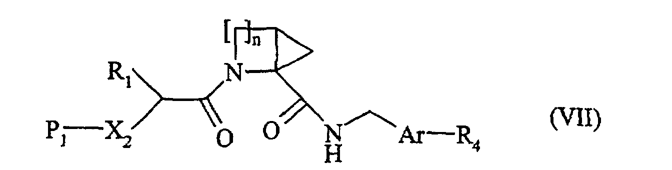 dans laquelle n, R₁, R₄, Ar, X₂ et P₁ ont la même signification que précédemment,
composé de formule (VII) que l'on transforme ensuite en composé de formule (I) par une ou plusieurs réactions de déprotection, d'alkylation ou d'amination réductrice, composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

Les composés de formule (II) sont obtenus selon le procédé décrit par A. Hercouet et coll. (Tetrahedron : Asymmetry 1996, Vol. 7 N°5, pp. 1267-1268 et Tet. Lett. 1996, Vol. 37 N°26, pp. 4529-4532).

Les composés de la présente invention, outre le fait qu'ils soient nouveaux, présentent des propriétés pharmacologiques particulièrement intéressantes.

Ce sont de puissants inhibiteurs de protéases à sérine apparentées à la trypsine qui présentent une importante sélectivité vis-à-vis de la thrombine par rapport à d'autres protéases à sérine de la coagulation et de la fibrinolyse.

Ces propriétés les rendent donc utiles dans le traitement des angines stables ou non, des maladies d'origine thrombotique et/ou donnant lieu à des complications thrombotiques, dans le traitement ou la prévention de l'infarctus du myocarde et des thromboses veineuses ou artérielles, ainsi que dans le traitement des complications des maladies vasculaires et cardiovasculaires telles que l'athérosclérose, l'artérite, la maladie veineuse, et dans le traitement de toutes les maladies impliquant une formation et/ou une activité de la thrombine.

Ils peuvent également être utilisés en association thérapeutique avec un thrombolytique.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Les abréviations utilisées dans les exemples sont :
- HOBT: 1-Hydroxybenzotriazole hydrate
- TBTU: Tétrafluoroborate de O-(1H-Benzotriazol-1-yl)-N,N,N',N'-tétraméthyluronium

Par (2*S*,3*R*)-2,3-méthano-proline, on entend acide (1*S*,5*R*)-2-azabicyclo[3.1.0]hexane-1-carboxylique. Par (2*S*,3*R*)-2,3-méthano-homoproline, on entend acide (1*S*,6*R*)-2-azabicyclo[4.1.0]heptane-1-carboxylique.

Par composé de configuration (3α) ou (3β), on entend composé choisi parmi les stéréoisomères (3*R*) et (3*S*), étant entendu que lorsque le composé (3α) représente l'un des stéréoisomères (3*R*) ou (3*S*), alors le composé (3β) représente l'autre stéréoisomère, la configuration absolue du carbone 3 n'étant pas définie.

Les produits de départ utilisés sont des produits de départ connus ou préparés selon des modes préparatoires connus.

Les préparations A à H conduisent à des intermédiaires de synthèse, utiles pour la préparation des composés de l'invention.

Les structures des composés décrits dans les exemples ont été déterminés selon les techniques spectrophotométriques usuelles (infrarouge, RMN, spectrométrie de masse,...).

### PREPARATION A : (2S,3R)-2,3-Méthano-prolinate de benzyle

Le produit attendu est obtenu selon le procédé décrit par A. Hercouet et coll. (Tetrahedron : Asymmetry 1996, Vol. 7 N° 5, pp. 1267-1268).

### PREPARATION B : (2S,3R)-2,3-Méthano-homoprolinate de benzyle

Le produit attendu est obtenu selon le procédé décrit par A. Hercouet et coll. (Tet. Lett. 1996, Vol. 37 N° 26, pp. 4529-4532).

### PREPARATION C : [4-Aminométhyl-phényl]-(imino)-méthyl-carbamate de benzyle

Le produit attendu est obtenu selon le procédé décrit par G. De Nanteuil et coll. (Synth. Comm. 1998, Vol. 28 N° 23, pp. 4419-4429).

### PREPARATION D : 6-Amino-3-aminométhylpyridine

A 10 mmoles de 6-aminonicotinamide dans le tétrahydrofurane est ajouté de l'hydrure de lithium aluminium (21 mmoles) par petites portions. Le mélange est porté au reflux pendant 72 heures, puis ramené à température ambiante, hydrolysé par de l'eau, puis par une solution de soude 1 N. Après 1 heure d'agitation, le mélange est filtré sur célite et lavé avec un mélange tétrahydrofurane/méthanol 85/15. Les solvants sont ensuite évaporés et le résidu obtenu est purifié par chromatographie sur gel de silice pour conduire au produit attendu sous la forme d'un solide blanc.

### PREPARATION E : 6-Amino-3-aminométhyl-2-méthylpyridine

### Stade A : 6-Amino-3-cyano-2-méthylpyridine

A 10 mmoles de 6-amino-3-bromo-2-méthylpyridine en solution dans le diméthylformamide est ajouté du cyanure de cuivre (I) (12 mmoles). Le mélange est porté au reflux pendant 10 heures, puis refroidi à 80°C et coulé dans une solution de cyanure de sodium (40 mmoles) dans l'eau. Après l'heure d'agitation à température ambiante, le mélange est extrait à l'acétate d'éthyle. La phase organique est lavée, puis séchée et évaporée pour conduire au produit attendu sous la forme d'un solide ocre.

### Stade B : 6-Amino-3-aminométhyl-2-méthylpyridine

Le produit attendu est obtenu selon le procédé décrit dans le stade B de l'exemple 1 à partir du composé décrit dans le stade précédent, en utilisant le nickel de Raney comme catalyseur.

### PREPARATION F : 6-Amino-3-aminométhyl-5-méthylpyridine

Le produit attendu est obtenu selon le procédé décrit dans la préparation E à partir de la 6-amino-3-bromo-5-méthylpyridine.

### PREPARATION G : [4-Aminométhyl-anilino]-(imino)-méthyl-carbamate de benzyle

### Stade A : 4-[(Amino-(imino)-méthyl)-amino]-benzylimidodicarbonate de di-(tert-butyle)

10 mmoles de 4-aminobenzylimidodicarbonate de di-(tert-butyle) et 100 mmoles de cyanamide sont portées à reflux dans 80 ml d'éthanol anhydre pendant 2 jours. Après évaporation de l'éthanol et purification par chromatographie sur silice, on obtient le produit attendu.

### Stade B : 4-[Di-(tert-butyloxycarbonyl)aminométhyl]-anilino-(imino)-méthyl-carbamate de benzyle

A 10 mmoles du composé obtenu dans le stade précédent en solution dans le tétrahydrofurane sont ajoutées 99 mmoles de soude 4N, puis, à 5°C et au goutte à goutte, 24 mmoles de chloroformate de benzyle. Après une nuit d'agitation à température ambiante, le mélange réactionnel est décanté, la phase organique est évaporée puis le résidu obtenu est lavé par de l'acétate d'éthyle, filtré puis séché pour conduire au produit attendu.

### Stade C : [4-Aminométhyl-anilino]-(imino)-méthyl-carbamate de benzyle

Un courant d'HC1 gaz est passé sous agitation, à 0°C, dans une solution du composé décrit dans le stade précédent (10 mmoles) dans l'acétate d'éthyle, pendant 30 minutes. Après 1 nuit d'agitation à température ambiante, le précipité formé est filtré, rincé à l'acétate d'éthyle puis séché sous vide au dessicateur.

### PREPARATION H : [4-Aminométhyl-anilino]-carbothioyl-carbamate de benzyle

Le produit attendu est obtenu selon le procédé décrit dans la préparation G à partir de 4-aminobenzylimidodicarbonate de di-(tert-butyle) et d'acide thiocyanique.

### EXEMPLE 1 : 1-[(2R)-3-Cyclohexylalanyl]-N-[(6-amino-2-méthyl-3-pyridyl)-méthyl]-(2S,3R)-2,3-méthano-prolinamide, dichlorhydrate

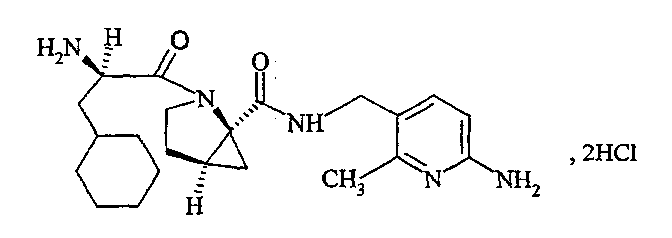

### Stade A : 1-[N-Tert-butyloxycarbonyl-(2R)-3-cyclohexylalanyl]-(2S,3R)-2,3-méthano-prolinate de benzyle

A une solution du composé décrit dans la préparation A (10 mmoles) et de *N-tert*-butyloxycarbonyl-(2*R*)-3-cyclohexylalanine (10 mmoles) dans le diméthylformamide sont ajoutés du TBTU (11 mmoles), de l'HOBT (11 mmoles) et de la diisopropyléthylamine (22 mmoles). Après une nuit d'agitation à température ambiante, le solvant est évaporé. Le résidu obtenu est repris par de l'acétate d'éthyle. La phase organique est lavée, puis elle est séchée et évaporée pour conduire au produit attendu sous forme d'huile.

### Stade B : 1-[N-Tert-butyloxycarbonyl-(2R)-3-cyclohexylalanyl]-(2S,3R)-2,3-méthano-proline

Une solution du composé décrit dans le stade précédent (10 mmoles) dans l'éthanol est placée sous hydrogène pendant une nuit en présence de Pd/C à 10 % (0,5 g). Après filtration du catalyseur, le solvant est évaporé pour conduire à un solide vitreux blanc.

### Stade C : 1-[N-Tert-butyloxycarbonyl-(2R)-3-cyclohexylalanyl]-N-[(6-amino-2-méthyl-3-pyridyl)-méthyl]-(2S,3R)-2,3-méthano-prolinamide

A une solution du composé décrit dans le stade précédent (10 mmoles) et du composé décrit dans la préparation E (10 mmoles) dans le diméthylformamide, sont ajoutés du TBTU (11 mmoles) et de la diisopropyléthylamine (11 mmoles). Après une nuit d'agitation à température ambiante, le solvant est évaporé. Le résidu obtenu est repris par de l'acétate d'éthyle. La phase organique est lavée, séchée puis évaporée. Le produit attendu est obtenu après purification du résidu par chromatographie sur gel de silice en utilisant comme éluant un mélange dichlorométhane/acétate d'éthyle (1/1).

### Stade D : 1-[(2R)-3-Cyclohexylalanyl]-N-[(6-amino-2-méthyl-3-pyridyl)-méthyl]-(2S,3R)-2,3-méthano-prolinamide, dichlorhydrate

Un courant d'HC1 gaz est passé sous agitation, à 0°C, dans une solution du composé décrit dans le stade précédent (10 mmoles) dans l'acétate d'éthyle, pendant 30 minutes. Après une nuit d'agitation à température ambiante, le précipité formé est filtré, rincé à l'acétate d'éthyle puis séché sous vide au dessicateur.

| Microanalyse élémentaire : | | | | |
|---|---|---|---|---|
| | *%C* | *%H* | *%N* | *%Cl* |
| Calculé | 55,93 | 7,47 | 14,82 | 15,01 |
| Trouvé | 55,93 | 7,68 | 14,10 | 14,47 |

Point de fusion : > 200°C

### EXEMPLE 2 : 1-[(2R)-3-Cyclohexylalanyl]-N-(4-amidinobenzyl)-(2S,3R)-2,3-méthano-prolinamide, dichlorhydrate

### Stade A : 1-[(2R)-3-Cyclohexylalanyl]-N-[4-(benzyloxycarbonylamino-(imino)-méthyl)-benzyl]-(2S,3R)-2,3-méthano-prolinamide, dichlorhydrate

Le produit attendu est obtenu selon le procédé décrit dans l'exemple 1 à partir du composé décrit dans la préparation A, de *N*-tert-butyloxycarbonyl-(2*R*)-3-cyclohexylalanine et du composé décrit dans la préparation C.

### Stade B : 1-[(2R)-3-Cyclohexylalanyl]-N-(4-amidinobenzyl)-(2S,3R)-2,3-méthano-prolinamide, dichlorhydrate

Le produit attendu est obtenu selon le procédé décrit dans le stade B de l'exemple 1, à partir du composé décrit dans le stade précédent.

| Microanalyse élémentaire : | | | | |
|---|---|---|---|---|
| | *%C* | *%H* | *%N* | *%Cl* |
| Calculé | 57,02 | 7,30 | 14,46 | 14,63 |
| Trouvé | 57,56 | 7,23 | 14,46 | 15,17 |

### EXEMPLE 3 : 1-[N-Carboxyméthyl-(2R)-3-cyclohexylalanyl]-N-(4-amidinobenzyl)-(2S,3R)-23-méthane-prolinamide, chlorhydrate

### Stade A : 1-[N-Benzylcarboxyméthyl-(2R)-3-cyclohexylalanyl]-N-(4-amidinobentyl) -(2S,3R)-2,3-méthano-prolinamide

A une solution du composé décrit dans l'exemple 2 (10 mmoles) dans l'acétonitrile est ajouté du carbonate de potassium (30 mmoles) puis du 2-bromoacétate de benzyle (11 mmoles). Après une nuit d'agitation, la solution est filtrée et évaporée, le résidu est repris par de l'acétate d'éthyle, la phase organique est lavée, séchée puis évaporée.

### Stade B : 1-(N-Carboxyméthyl-(2R)-3-cyclohexylalanyl]-N-(4-amidinobenzyl)-(2S,3R)-2,3-méthano-prolinamide

Le produit attendu est obtenu selon le procédé décrit dans le stade B de l'exemple 1, à partir du composé obtenu dans le stade précédent.

### Stade C : 1-[N-Carboxyméthyl-(2R)-3-cyclohexylalanyl]-N-(4-amidinobenzyl)-(2S, 3R)-2,3-méthano-prolinamide, chlorhydrate

Le produit attendu est obtenu selon le procédé décrit dans le stade D de l'exemple 1, à partir du composé obtenu dans le stade précédent.

| Microanalyse élémentaire | | | | |
|---|---|---|---|---|
| | *%C* | *%H* | *%N* | *%Cl* |
| Calculé | 59,34 | 7,17 | 13,84 | 7,01 |
| Trouvé | 59,43 | 7,16 | 13,73 | 7,60 |

### EXEMPLE 4 : 1-[N-Méthyl-(2R)-3-cyclohexylalanyl]-N-(4-isothiouréïdobenzyl)-(2S,3R)-2,3-méthano-prolinamide, dichlorhydrate

### Stade A : 1-[(2R)-3-Cyclohexylalanyl]-N-(4-isothiouréïdobenzyl)-(2S,3R)-2,3-méthano-prolinamide, dichlorhydrate

Le produit attendu est obtenu selon le procédé décrit dans l'exemple 2, à partir du composé décrit dans la préparation A, de *N*-tert-butyloxycarbonyl-(2*R*)-3-cyclohexylalanine et du composé décrit dans la préparation H.

### Stade B : 1-[N-Méthyl-(2R)-3-cyclohexylalanyl]-N-(4-isothiouréïdobenzyl)-(2S,3R)-2,3-méthano-prolinamide

Le produit attendu est obtenu selon le procédé décrit dans J. Org. Chem. 1996, 61, 3849-3862 à partir du composé obtenu dans le stade précédent et de formaldéhyde, en présence de triacétoxyborohydrure de sodium.

### Stade C : 1-[N-Méthyl-(2R)-3-cyclohexylalanyl]-N-(4-isothiouréïdobenzyl)-(2S,3R)-2,3-méthane-prolinamide, dichlorhydrate

Le produit attendu est obtenu selon le procédé décrit dans le stade D de l'exemple 1, à partir du composé décrit dans le stade précédent.

### EXEMPLE 5: 1-[N-Benzyl-(2R)-3-cyclohexylalanyl]-N-(4-isothiouréïdobenzyl)-(2S,3R)-2,3-méthano-prolinamide, dichlorhydrate

### Stade A : 1-[N-Benzyl-(2R)-3-cyclohexylalanyl]-N-(4-isothiouréïdobenzyl)-(2S,3R)-2,3-méthane-prolinamide

A une solution à 0°C du composé obtenu dans le stade A de l'exemple 4 (10 mmoles) dans le dichlorométhane est ajoutée de la diisopropyléthylamine (30 mmoles), puis, au goutte à goutte, du chlorure de benzyle (11 mmoles). Après 4 heures d'agitation à 0°C, la solution est hydrolysée, la phase organique est lavée par une solution d'acide chlorhydrique 1N puis par de l'eau, puis elle est évaporée. Le produit attendu est obtenu après purification du résidu par chromatographie sur gel de silice en utilisant comme éluant un mélange dichlorométhane/acétate d'éthyle.

### Stade B : 1-[N-Benzyl-(2R)-3-cyclohexylalanyl]-N-(4-isothiouréïdobenzyl)-(2S,3R)-2,3-méthano-prolinamide, dichlorhydrate

Le produit attendu est obtenu selon le procédé décrit dans le stade D de l'exemple 1, à partir du composé décrit dans le stade précédent.

### EXEMPLE 6 : 1-[N-Méthyl-(2R)-3-cyclohexylalanyl]-N-(4-guanidinobenyl)-(2S,3R)-2,3-méthano-prolinamide, dichlorhydrate

### Stade A : 1-[(2R)-3-Cyclohexylalanyl]-N-(4-guanidinobenzyl)-(2S,3R)-2,3-méthano-prolinamide, dichlorhydrate

Le produit attendu est obtenu selon le procédé décrit dans l'exemple 2 à partir du composé décrit dans la préparation A, de *N*-tert-butyloxycarbonyl-(2*R*)-3-cyclohexylalanine et du composé décrit dans la préparation G.

### Stade B : 1-[N-Méthyl-(2R)-3-cyclohexylalanyl]-N-(4-guanidinobenzyl)-(2S,3R)-2,3-méthano-prolinamide, dichlorhydrate

Le produit attendu est obtenu selon le procédé décrit dans les stades B et C de l'exemple 4 à partir du composé obtenu dans le stade précédent.

### EXEMPLE 7: 1-[N-Benzyl-(2R)-3-cyclohexylalanyl]-N-(4-guanidinobenzyl)-(2S,3R)-2,3-méthano-prolinamide, dichlorhydrate

Le produit attendu est obtenu selon le procédé décrit dans l'exemple 5 à partir du composé obtenu dans le stade A de l'exemple 6.

### EXEMPLE 8 : 1-[(2R)-3-Phénylalanyl]-N-(4-amidinobenzyl)-(2S,3R)-2,3-méthano-prolinamide, dichlorhydrate

Le produit attendu est obtenu selon le procédé décrit dans l'exemple 2, à partir du composé décrit dans la préparation A, de *N*-tert-butyloxycarbonyl-(2*R*)-3-phénylalanine et du composé décrit dans la préparation C.

| Microanalyse élémentaire | | | | |
|---|---|---|---|---|
| | *%C* | *%H* | *%N* | *%Cl* |
| Calculé | 57,74 | 6,11 | 14,64 | 14,82 |
| Trouvé | 57,64 | 5,91 | 14,45 | 14,65 |

### EXEMPLE 9 : 1-[N-Benzylsulfonyl-(2R)-3-phénylalanyl]-N-(4-amidinobenzyl)-(2S,3R)-2,3-méthano-prolinamide, chlorhydrate

### Stade A : 1-[N-Benzylsulfonyl-(2R)-3-phénylalanyl]-N-(4-amidinobenzyl)-(2S,3R)-2,3-méthano-prolinamide

A une solution à 0°C du composé décrit dans l'exemple 8 (10 mmoles) dans le dichlorométhane est ajoutée de la diisopropyléthylamine (30 mmoles) puis, au goutte à goutte, du chlorure de phénylméthanesulfonyle (11 mmoles). Après 4 heures d'agitation à 0°C, la solution est hydrolysée, la phase organique est lavée par une solution d'acide chlorhydrique 1 N puis par de l'eau, puis elle est évaporée. Le produit attendu est obtenu après purification du résidu par chromatographie sur gel de silice en utilisant comme éluant un mélange dichlorométhane/acétate d'éthyle.

### Stade B : 1-[N-Benzylsulfonyl-(2R)-3-phénylalanyl]-N-(4-amidinobenzyl)-(2S,3R)-2,3-méthano-prolinamide, chlorhydrate

Le produit attendu est obtenu selon le procédé décrit dans le stade D de l'exemple 1, à partir du composé décrit dans le stade précédent.

| Microanalyse élémentaire | | | | | |
|---|---|---|---|---|---|
| | *%C* | *%H* | *%N* | *%Cl* | *%S* |
| Calculé | 60,44 | 5,75 | 11,75 | 5,95 | 5,38 |
| Trouvé | 60,90 | 5,67 | 11,50 | 6,11 | 5,11 |

### EXEMPLE 10 : 1-[N-Carboxyméthyl-(2R)-3-phénylalanyl)-N-(4-amidinobenzyl)-(2S,3R)-2,3-méthano-prolinamide, chlorhydrate

Le produit attendu est obtenu selon le procédé décrit dans l'exemple 3, à partir du composé décrit dans l'exemple 8.

| Microanalyse élémentaire | | | | |
|---|---|---|---|---|
| | *%C* | *%H* | *%N* | *%Cl* |
| Calculé | 57,13 | 5,90 | 13,33 | 11,47 |
| Trouvé | 57,65 | 5,51 | 13,40 | 11,51 |

### EXEMPLE 11 : 1-[(2R)-3,3-Diphénylalanyl]-N-(4-amidinobenzyl)-(2S,3R)-2,3-méthano-prolinamide, dichlorhydrate

Le produit attendu est obtenu selon le procédé décrit dans l'exemple 2, à partir du composé décrit dans la préparation A, de *N*-tert-butyloxycarbonyl-(2R)-3,3-diphénylalanine et du composé décrit dans la préparation C.

| Microanalyse élémentaire | | | | |
|---|---|---|---|---|
| | *%C* | *%H* | *%N* | *%Cl* |
| Calculé | 62,81 | 6,00 | 12,63 | 12,79 |
| Trouvé | 63,12 | 6,02 | 12,71 | 13,22 |

### EXEMPLE 12 : 1-[(2R)-3,3-Diphénylalanyl]-N-[(6-amino-3-pyridyl)-méthyl]-(2S,3R)-2,3-méthane-prolinamide, dichlorhydrate

Le produit attendu est obtenu selon le procédé décrit dans l'exemple 1, à partir du composé décrit dans la préparation A, de *N*-tert-butyloxycarbonyl-(2*R*)-3,3-diphénylalanine et du composé décrit dans la préparation D.

| Microanalyse élémentaire | | | | |
|---|---|---|---|---|
| | *%C* | *%H* | *%N* | *%Cl* |
| Calculé | 61,36 | 5,91 | 13,25 | 13,42 |
| Trouvé | 61,98 | 6,04 | 13,46 | 13,67 |

### EXEMPLE 13 : 1-[(2R)-3,3-Dicyclohexylalanyl]-N-(4-amidinobenzyl)-(2S,3R)-2,3-méthano-prolinamide, dichlorhydrate

### Stade A : N-Tert-butyloxycarbonyl-(2R)-3,3-dicyclohexylalanine

Une solution de *N-tert*-butyloxycarbonyl-(2*R*)-3,3-diphénylalanine (10 mmoles) dans l'éthanol est placée sous hydrogène pendant une nuit en présence de Rh/C (0,5 g). Après filtration du catalyseur, le solvant est évaporé pour conduire au produit attendu.

### Stade B : 1-[(2R)-3,3-Dicyclohexylalanyl]-N-(4-amidinobenzyl)-(2S,3R)-2,3-méthano-prolinamide, dichlorhydrate

Le produit attendu est obtenu selon le procédé décrit dans l'exemple 2, à partir du composé décrit dans la préparation A, du composé décrit dans le stade précédent et du composé décrit dans la préparation C.

| Microanalyse élémentaire | | | | |
|---|---|---|---|---|
| | *%C* | *%H* | *%N* | *%Cl* |
| Calculé | 61,47 | 8,00 | 12,36 | 12,51 |
| Trouvé | 61,88 | 7,89 | 12,32 | 12,46 |

### EXEMPLE 14 : 1-[N-Carboxyméthyl-(2R)-3,3-dicyclohexylalanyl]-N-(4-amidinobenzyl)-(2S,3R)-2,3-méthano-prolinamide, chlorhydrate

Le produit attendu est obtenu selon le procédé décrit dans l'exemple 3, à partir du composé décrit dans l'exemple 13.

| Microanalyse élémentaire | | | | |
|---|---|---|---|---|
| | *%C* | *%H* | *%N* | *%Cl* |
| Calculé | 63,30 | 7,88 | 11,91 | 6,03 |
| Trouvé | 63,78 | 7,50 | 11,81 | 6,21 |

### EXEMPLE 15 : 1-[(2R)-Cyclohexylglycyl]-N-(4-amidinobenzyl)-(2S,3R)-2,3-méthano-prolinamide, dichlorhydrate

Le produit attendu est obtenu selon le procédé décrit dans l'exemple 2, à partir du composé décrit dans la préparation A, de *N*-tert-butyloxycarbonyl-(2*R*)-cyclohexylglycine et du composé décrit dans la préparation C.

### EXEMPLE 16: 1-[N-Benzylsulfonyl-(2R)-cyclohexylglycyl]-N-(4-amidinobenzyl)-(2S,3R)-2,3-méthano-prolinamide, chlorhydrate

Le produit attendu est obtenu selon le procédé décrit dans l'exemple 9, à partir du composé décrit dans l'exemple 15.

| Microanalyse élémentaire : | | | | | |
|---|---|---|---|---|---|
| | *%C* | *%H* | *%N* | *%Cl* | *%S* |
| Calculé | 59,22 | 6,51 | 11,91 | 6,03 | 5,45 |
| Trouvé | 59,31 | 6,37 | 11,93 | 6,49 | 5,26 |

### EXEMPLE 17 : 1-[N-Carboxyméthyl-(2R)-cyclohexylglycyl]-N-(4-amidinobenzyl)-(2S,3R)-2,3-méthano-prolinamide, chlorhydrate

Le produit attendu est obtenu selon le procédé décrit dans l'exemple 3, à partir du composé décrit dans l'exemple 15.

| Microanalyse élémentaire : | | | | |
|---|---|---|---|---|
| | *%C* | *%H* | *%N* | *%Cl* |
| Calculé | 58,59 | 6,96 | 14,23 | 7,51 |
| Trouvé | 59,15 | 6,53 | 14,14 | 8,48 |

### EXEMPLE 18 : 1-[N-Ethoxycarbonylméthyl-(2R)-cyclohexylglycyl]-N-(4-amidinobenzyl)-(2S,3R)-2,3-méthano-prolinamide, dichlorhydrate

Le produit attendu est obtenu selon le procédé décrit dans l'exemple 9, à partir de bromoacétate d'éthyle et du composé décrit dans l'exemple 15.

| Microanalyse élémentaire : | | | | |
|---|---|---|---|---|
| | *%C* | *%H* | *%N* | *%Cl* |
| Calculé | 56,11 | 7,06 | 12,58 | 12,74 |
| Trouvé | 56,92 | 7,18 | 12,81 | 12,78 |

### EXEMPLE 19 : 1-[N-Carboxyméthyl-(2R)-leucyl]-N-(4-amidinobenzyl)-(2S,3R)-2,3-méthano-prolinamide, chlorhydrate

### Stade A : 1-[(2R)-Leucyl]-N-(4-amidinobenzyl)-(2S,3R)-2,3-méthano-prolinamide, dichlorhydrate

Le produit attendu est obtenu selon le procédé décrit dans l'exemple 2 à partir du composé décrit dans la préparation A, de *N*-tert-butyloxycarbonyl-(2*R*)-leucine et du composé décrit dans la préparation C.

### Stade B : 1-[N-Carboxyméthyl-(2R)-leucyl]-N-(4-amidinobenzyl)-(2S,3R)-2,3-méthano-prolinamide, dichlorhydrate

Le produit attendu est obtenu selon le procédé décrit dans l'exemple 3 à partir du composé décrit dans le stade précédent.

| Microanalyse élémentaire | | | | |
|---|---|---|---|---|
| | *%C* | *%H* | *%N* | *%Cl* |
| Calculé | 56,71 | 6,92 | 15,03 | 7,61 |
| Trouvé | 56,63 | 7,02 | 14,81 | 8,20 |

### EXEMPLE 20 : 1-[N-Méthyl-(2R)-phénylglycyl]-N-(4-amidinobenzyl)-(2S,3R)-2,3-méthano-prolinamide, dichlorhydrate

### Stade A : 1-[(2R)-2-Phénylglycyl]-N-(4-amidinobenzyl)-(2S,3R)-2,3-méthano-prolinamide, dichlorhydrate

Le produit attendu est obtenu selon le procédé décrit dans l'exemple 2 à partir du composé décrit dans la préparation A, de *N*-tert-butyloxycarbonyl-(2R)-phénylglycine et du composé décrit dans la préparation C.

### Stade B : 1-[N-Méthyl-(2R)-phénylglycyl]-N-(4-amidinobenzyl)-(2S,3R)-2,3-méthano-prolinamide, dichlorhydrate

Le produit attendu est obtenu selon le procédé décrit dans les stades B et C de l'exemple 4 à partir du composé décrit dans le stade précédent.

### EXEMPLE 21 : 1-[N-Benzyl-(2R)-phénylglycyl]-N-(4-amidinobenzyl)-(2S,3R)-2,3-méthano-prolinamide, dichlorhydrate

Le produit attendu est obtenu selon le procédé décrit dans l'exemple 5, à partir du composé décrit dans le stade A de l'exemple 20.

### EXEMPLE 22 : 1-[(2R)-3-Cyclohexyl-2-hydroxy-propionyl]-N-(4-amidinobenzyl)-(2S,3R)-2,3-méthano-prolinamide, chlorhydrate

### Stade A : Acide (2R)-3-cyclohexyl-2-hydroxypropionique

Le produit attendu est obtenu selon le procédé décrit dans le stade A de l'exemple 13 à partir de l'acide (2*R*)-2-hydroxy-3-phénylpropionique.

### Stade B : Acide (2R)-2-acétoxy-3-cyclohexyl-propionique

A 10 mmoles du composé obtenu dans le stade précédent en solution dans la pyridine est ajouté de l'anhydride acétique (11 mmoles). Après 1 heure d'agitation à température ambiante, le mélange réactionnel est versé dans l'eau et du méthyl-*tert*-butyl-éther est ajouté. La phase organique est lavée, séchée puis évaporée pour conduire au produit attendu.

### Stade C : 1-[(2R)-2-Acétoxy-3-cyclohexyl-propionyl]-N-[4-(benzyloxy-carbonylamino-(imino)-méthyl)-benzyl]-(2S,3R)-2,3-méthano-prolinamide

Le produit attendu est obtenu selon le procédé décrit dans les stades A à C de l'exemple 1, à partir du composé décrit dans la préparation A, du composé décrit dans le stade précédent et du composé décrit dans la préparation C.

### Stade D : 1-[(2R)-3-Cyclohexyl-2-hydroxy-propionyl]-N-[4-(benzyloxy-carbonylamino-(imino)-méthyl)-benzyl]-(2S,3R)-2,3-méthane-prolinamide

A 10 mmoles du composé décrit dans le stade précédent en solution dans le tétrahydrofurane est ajoutée une solution de soude 1 N. Après 1 heure d'agitation à température ambiante, de l'acétate d'éthyle est ajouté, la phase organique est lavée, séchée puis évaporée pour conduire au produit attendu.

### Stade E : 1-[(2R)-3-Cyclohexyl-2-hydroxy-propionyl]-N-(4-amidinobenzyl)-(2S,3R)-2,3-méthano-prolinamide, chlorhydrate

Une solution du composé décrit dans le stade précédent (10 mmoles) dans l'éthanol chlorhydrique 1 N est placée sous hydrogène pendant une nuit en présence de Pd/C à 10 % (0,5 g). Après filtration du catalyseur, le solvant est évaporé pour conduire au produit attendu.

| Microanalyse élémentaire | | | | |
|---|---|---|---|---|
| | *%C* | *%H* | *%N* | *%Cl* |
| Calculé | 61,53 | 7,41 | 12,48 | 7,90 |
| Trouvé | 61,36 | 7,40 | 12,45 | 8,47 |

### EXEMPLE 23 : 1-[(2R)-3-Cyclohexylalanyl]-N-(4-amidinobenzyl)-(2S,3R)-2,3-méthano-homoprolinamide, dichlorhydrate

Le produit attendu est obtenu selon le procédé décrit dans l'exemple 2, à partir du composé décrit dans la préparation B, de *N*-tert-butyloxycarbonyl-(2*R*)-3-cyclohexylalanine et du composé décrit dans la préparation C.

### EXEMPLE 24 : 1-[N-Carboxyméthyl-(2R)-3-cyclohexylalanyl]-N-(4-amidinobenzyl)-(2S,3R)-2,3-méthano-homoprolinamide, chlorhydrate

Le produit attendu est obtenu selon le procédé décrit dans l'exemple 3, à partir du composé décrit dans l'exemple 23.

| Microanalyse élémentaire | | | | |
|---|---|---|---|---|
| | *%C* | *%H* | *%N* | *%Cl* |
| Calculé | 60,05 | 7,36 | 13,47 | 6,82 |
| Trouvé | 59,52 | 7,18 | 12,70 | 7,67 |

### EXEMPLE 25 : 1-[N-Ethoxycarbonylméthyl-(2R)-3-cyclohexylalanyl]-N-(4-amidinobenzyl)-(2S,3R)-2,3-méthano-homoprolinamide, dichlorhydrate

Le produit attendu est obtenu selon le procédé décrit dans l'exemple 9, à partir de bromoacétate d'éthyle et du composé décrit dans l'exemple 23.

| Microanalyse élémentaire | | | | |
|---|---|---|---|---|
| | *%C* | *%H* | *%N* | *%Cl* |
| Calculé | 57,53 | 7,41 | 11,98 | 12,13 |
| Trouvé | 58,29 | 7,43 | 11,89 | 11,75 |

### EXEMPLE 26 : 1-[N-Carbamoylméthyl-(2R)-3-cyclohexylalanyl]-N-(4-amidino-benzyl)-(2S,3R)-2,3-méthano-homoprolinamide, dichlorhydrate

Le produit attendu est obtenu selon le procédé décrit dans l'exemple 9, à partir du bromo-2-acétamide et du composé décrit dans l'exemple 23.

### EXEMPLE 27 : 1-[(2R)-3-Cyclohexylalanyl]-N-[(6-amino-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3-cyclohexylalanine et du composé décrit dans la préparation D.

| Microanalyse élémentaire : | | | | |
|---|---|---|---|---|
| | *%C* | *%H* | *%N* | *%Cl* |
| Calculé | 55,93 | 7,47 | 14,82 | 15,01 |
| Trouvé | 55,91 | 7,38 | 14,67 | 15,33 |

### EXEMPLE 28 : 1-[(2R)-3-Cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3-cyclohexylalanine et du composé décrit dans la préparation E.

| Microanalyse élémentaire | | | | |
|---|---|---|---|---|
| | *%C* | *%H* | *%N* | *%Cl* |
| Calculé | 56,79 | 7,67 | 14,40 | 14,57 |
| Trouvé | 57,37 | 7,87 | 14,21 | 14,82 |

### EXEMPLE 29 : 1-[N-Méthyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans les stades B et C de l'exemple 4, à partir du composé décrit dans l'exemple 28.

| Microanalyse élémentaire : | | | | |
|---|---|---|---|---|
| | *%C* | *%H* | *%N* | *%Cl* |
| Calculé | 57,59 | 7,85 | 13,99 | 14,17 |
| Trouvé | 57,63 | 7,53 | 13,95 | 14,66 |

### EXEMPLE 30 : 1-[N-Ethyl-(2R)-3-cyclohexylalanyll-N-[(6-amino-2-méthyl-3-pyridyl)-méthyl]-(2S,3R)-2,3-méthano-homoprolinamide, dichlorhydrate

### Stade A : 1-[N-Ethyl-(2R)-3-cyclohexylalanyl]-N-[(6-amino-2-méthyl-3-pyridyl)-méthyl]-(2S,3R)-2,3-méthano-homoprolinamide

Le produit attendu est obtenu selon le procédé décrit dans J. Org. Chem., 1996, 61, 3849-3862 à partir du composé décrit dans l'exemple 28 et d'acétaldéhyde, en présence de triacétoxyborohydrure de sodium.

### Stade B : 1-[N-Ethyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans le stade D de l'exemple 1 à partir du composé décrit dans le stade précédent.

### EXEMPLE 31 : 1-[N-Butyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et de butyraldéhyde.

| Microanalyse élémentaire | | | | |
|---|---|---|---|---|
| | *%C* | *%H* | *%N* | *%Cl* |
| Calculé | 59,77 | 8,36 | 12,91 | 13,07 |
| Trouvé | 60,14 | 8,25 | 12,83 | 13,29 |

### EXEMPLE 32 : 1-[N-Carboxyméthyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 3, à partir du composé décrit dans l'exemple 28.

| Microanalyse élémentaire : | | | | |
|---|---|---|---|---|
| | *%C* | *%H* | *%N* | *%Cl* |
| Calculé | 55,14 | 7,23 | 12,86 | 13,02 |
| Trouvé | 55,73 | 7,24 | 12,68 | 13,00 |

### EXEMPLE 33 : 1-[N-Ethoxycarbonylméthyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir du bromoacétate d'éthyle et du composé décrit dans l'exemple 28.

| Microanalyse élémentaire : | | | | |
|---|---|---|---|---|
| | *%C* | *%H* | *%N* | *%Cl* |
| Calculé | 56,64 | 7,57 | 12,07 | 12,35 |
| Trouvé | 56,55 | 7,58 | 12,23 | 12,38 |

### EXEMPLE 34 : 1-[N-Ethoxycarbonylméthoxycarbonylméthyl-(2R)-3-cyclohexyl-alanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir de bromoacétate d'éthyle et du composé décrit dans l'exemple 32.

| Microanalyse élémentaire : | | | | |
|---|---|---|---|---|
| | *%C* | *%H* | *%N* | *%CI* |
| Calculé | 55,24 | 7,19 | 11,11 | 11,24 |
| Trouvé | 55,60 | 7,23 | 11,29 | 11,34 |

### EXEMPLE 35 : 1-[N-(Carbamoylméthyl)-(2R)-3-cyclohezylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir du bromo-2-acétamide et du composé décrit dans l'exemple 28.

| Microanalyse élémentaire : | | | | |
|---|---|---|---|---|
| | *%C* | *%H* | *%N* | *%Cl* |
| Calculé | 55,24 | 7,42 | 15,46 | 13,05 |
| Trouvé | 55,66 | 7,25 | 15,41 | 12,98 |

### EXEMPLE 36 : 1-[N-Benzyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 5, à partir du composé décrit dans l'exemple 28.

| Microanalyse élémentaire : | | | | |
|---|---|---|---|---|
| | *%C* | *%H* | *%N* | *%Cl* |
| Calculé | 62,49 | 7,52 | 12,15 | 12,30 |
| Trouvé | 62,38 | 7,64 | 11,47 | 11,52 |

### EXEMPLE 37 : 1-[N-(4-Hydroxybenzyl)-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et de 4-acétoxy-benzaldéhyde.

| Microanalyse élémentaire : | | | | |
|---|---|---|---|---|
| | *%C* | *%H* | *%N* | *%Cl* |
| Calculé | 60,80 | 7,31 | 11,82 | 11,96 |
| Trouvé | 60,20 | 7,59 | 11,35 | 11,61 |

### EXEMPLE 38 : 1-[N-(4-Carboxybenzyl)-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et de 4-(méthoxycarbonyl)-benzaldéhyde.

| Microanalyse élémentaire | | | | |
|---|---|---|---|---|
| | *%C* | *%H* | *%N* | *%Cl* |
| Calculé | 60,00 | 6,98 | 11,28 | 11,43 |
| Trouvé | 60,55 | 7,14 | 11,37 | 11,12 |

### EXEMPLE 39 : 1-[N-(3,4-Diméthoxybenzyl)-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et de (3,4-diméthoxy)-benzaldéhyde.

| Microanalyse élémentaire | | | | |
|---|---|---|---|---|
| | *%C* | *%H* | *%N* | *%Cl* |
| Calculé | 60,37 | 7,44 | 10,67 | 10,73 |
| Trouvé | 60,49 | 7,47 | 11,00 | 11,14 |

### EXEMPLE 40 : 1-[N-[(Biphénylyl)-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du 4-biphénylecarbaldéhyde.

| Microanalyse élémentaire | | | | |
|---|---|---|---|---|
| | *%C* | *%H* | *%N* | *%Cl* |
| Calculé | 66,25 | 7,26 | 10,73 | 10,86 |
| Trouvé | 66,65 | 7,17 | 10,75 | 11,10 |

### EXEMPLE 41 : 1-[N-[(2-Naphtyl)-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du 2-naphtalènecarbaldéhyde.

| Microanalyse élémentaire | | | | |
|---|---|---|---|---|
| | *%C* | *%H* | *%N* | *%Cl* |
| Calculé | 65,17 | 7,24 | 11,18 | 11,31 |
| Trouvé | 65,49 | 7,42 | 10,84 | 10,64 |

### EXEMPLE 42 : 1-[N-Phénéthyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du phénylacétaldéhyde.

| Microanalyse élémentaire : | | | | |
|---|---|---|---|---|
| | *%C* | *%H* | *%N* | *%Cl* |
| Calculé | 63,04 | 7,68 | 11,86 | 12,00 |
| Trouvé | 63,28 | 7,62 | 11,73 | 12,01 |

### EXEMPLE 43 : 1-[N-[(4-Pyridyl)-méthyl]-(2R)-3-cyclohexylalanyl]-N-[(6-amino-2-méthyl-3-pyridyl)-méthyl]-(2S,3R)-2,3-méthano-homoprolinamide, trichlorhydrate

Le produit attendu est obtenu selon le procédé décrit dans l'exemple 30 à partir du composé décrit dans l'exemple 28 et de l'isonicotinaldéhyde.

| Microanalyse élémentaire : | | | | |
|---|---|---|---|---|
| | *%C* | *%H* | *%N* | *%Cl* |
| Calculé | 56,72 | 7,06 | 13,69 | 17,32 |
| Trouvé | 56,30 | 7,14 | 13,37 | 18,10 |

### EXEMPLE 44 : 1-[N-[(2-Pyridyl)-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du 2-pyridinecarbaldéhyde.

| Microanalyse élémentaire : | | | | |
|---|---|---|---|---|
| | *%C* | *%H* | *%N* | *%Cl* |
| Calculé | 60,31 | 7,33 | 14,55 | 12,28 |
| Trouvé | 60,70 | 7,33 | 14,52 | 12,85 |

### EXEMPLE 45 : 1-[N-[(4-Quinolyl)-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du 4-quinoléinecarbaldéhyde.

| Microanalyse élémentaire : | | | | |
|---|---|---|---|---|
| | *%C* | *%H* | *%N* | *%Cl* |
| Calculé | 63,14 | 7,08 | 13,39 | 11,30 |
| Trouvé | 63,38 | 6,93 | 13,40 | 11,79 |

### EXEMPLE 46 : 1-[N-[(2-Quinolyl)-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 30 à partir du composé décrit dans l'exemple 28 et du 2-quinoléinecarbaldéhyde.

| Microanalyse élémentaire : | | | | |
|---|---|---|---|---|
| | *%C* | *%H* | *%N* | *%Cl* |
| Calculé | 63,14 | 7,08 | 13,39 | 11,30 |
| Trouvé | 62,70 | 7,14 | 13,53 | 11,67 |

### EXEMPLE 47 : 1-[N-[(4,5-Dihydro-1H-2-imidazoly]-méthyl]-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9 à partir du composé décrit dans l'exemple 28 et de 2-(chlorométhyl)-4,5-dihydro-1*H*-imidazole.

| Microanalyse élémentaire : | | | | |
|---|---|---|---|---|
| | *%C* | *%H* | *%N* | *%Cl* |
| Calculé | 57,04 | 7,62 | 17,24 | 12,47 |
| Trouvé | 57,88 | 7,56 | 17,19 | 12,64 |

### EXEMPLE 48 : 1-[N-Propargyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir du bromure de propargyle et du composé décrit dans l'exemple 28.

### EXEMPLE 49 : 1-[N-((4-Morpholinyl)-éthyl)-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir de la 4-(2-chloroéthyl)-morpholine et du composé décrit dans l'exemple 28.

### EXEMPLE 50 : 1-[N-((2-Morpholinyl)-méthyl)-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir de la 2-p-toluènesulfonyloxyméthyl-morpholine et du composé décrit dans l'exemple 28.

### EXEMPLE 51 : 1-[N-((4-Morpholinyl)-carbonyl)-(2R)-3-cyclohexylalanyl]-N-[(6-amino-2-méthyl-3-pyridyl)-méthyl]-(2S,3R)-2,3-méthano-homoprolinamide, chlorhydrate

Le produit attendu est obtenu selon le procédé décrit dans l'exemple 9, à partir du chlorure de 4-morpholine-carbonyle et du composé décrit dans l'exemple 28.

### EXEMPLE 52 : 1-[N-Méthylsulfonyl-(2R)-3-cyclohexylalanyl]-N-[(6-amino-2-méthyl-3-pyridyl)-méthyl]-(2S,3R)-2,3-méthano-homoprolinamide, chlorhydrate

Le produit attendu est obtenu selon le procédé décrit dans l'exemple 9, à partir du composé décrit dans l'exemple 28 et de chlorure de méthanesulfonyle.

### EXEMPLE 53 : 1-[N-Benzylsulfonyl-(2R)-3-cyclohexylalanyl]-N-[(6-amine-2-méthyl-3-pyridyl)-méthyl]-(2S,3R)-2,3-méthano-homoprolinamide, chlorhydrate

Le produit attendu est obtenu selon le procédé décrit dans l'exemple 9, à partir du composé décrit dans l'exemple 28.

### EXEMPLE 54 : 1-[N-Aminosulfonylméthyl-(2R)-3-cyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 9, à partir du composé décrit dans l'exemple 28 et de bromure d'aminosulfonylméthyle.

### EXEMPLE 55 : 1-[(2R)-3-Cyclohexylalanyl]-N-[(6-amino-5-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3-cyclohexylalanine et du composé décrit dans la préparation F.

| Microanalyse élémentaire | | | | |
|---|---|---|---|---|
| | *%C* | *%H* | *%N* | *%Cl* |
| Calculé | 56,79 | 7,67 | 14,40 | 14,58 |
| Trouvé | 57,26 | 7,71 | 14,28 | 15,08 |

### EXEMPLE 56 : 1-[N-Méthyl-(2R)-3-cyclohexylalanyl)-N-(4-isothiouréïdobenzyl)-2S,3R)-2,3-méthano-homoprolinamide, dichlorhydrate

Le produit attendu est obtenu selon le procédé décrit dans l'exemple 4 à partir du composé décrit dans la préparation B, de *N*-tert-butyloxycarbonyl-(2*R*)-3-cyclohexylalanine et du composé décrit dans la préparation H.

### EXEMPLE 57: 1-[N-Benzyl-(2R)-3-cyclohexylalanyl)-N-(4-isothiouréïdobenzyl)-(2S,3R)-2,3-méthano-homoprolinamide, dichlorhydrate

Le produit attendu est obtenu selon le procédé décrit dans l'exemple 5 à partir du composé décrit dans la préparation B, de *N*-tert-butyloxycarbonyl-(2R)-3-cyclohexylalanine et du composé décrit dans la préparation H.

### EXEMPLE 58 : 1-[N-Méthyl-(2R)-3-cyclohexylalanyl]-N-(4-guanidinobenzyl)-(2S,3R)-2,3-méthano-homoprolinamide, dichlorhydrate

Le produit attendu est obtenu selon le procédé décrit dans l'exemple 4 à partir du composé décrit dans la préparation B, de *N*-tert-butyloxycarbonyl-(2*R*)-3-cyclohexylalanine et du composé décrit dans la préparation G.

### EXEMPLE 59: 1-[N-Benzyl-(2R)-3-cyclohexylalanyl]-N-(4-guanidinobenzyl)-(2S,3R)-2,3-méthano-homoprolinamide, dichlorhydrate

Le produit attendu est obtenu selon le procédé décrit dans l'exemple 5 à partir du composé décrit dans la préparation B, de *N*-tert-butyloxycarbonyl-(2R)-3-cyclohexylalanine et du composé décrit dans la préparation G.

### EXEMPLE 60 : 1-[(2R)-3-Phénylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3-phénylalanine et du composé décrit dans la préparation E.

| Microanalyse élémentaire | | | | |
|---|---|---|---|---|
| | *%C* | *%H* | *%N* | *%Cl* |
| Calculé | 57,50 | 6,50 | 14,58 | 14,76 |
| Trouvé | 57,52 | 6,44 | 14,21 | 14,98 |

### EXEMPLE 61 : 1-[(2R)-3,3-Diphénylalanyl]-N-(4-amidinobenzyl)-(2S,3R)-2,3-méthano-homoprolinamide, dichlorhydrate

Le produit attendu est obtenu selon le procédé décrit dans l'exemple 2, à partir du composé décrit dans la préparation B, de *N*-tert-butyloxycarbonyl-(2*R*)-3,3-diphénylalanine et du composé décrit dans la préparation C.

| Microanalyse élémentaire | | | | |
|---|---|---|---|---|
| | *%C* | *%H* | *%N* | *%Cl* |
| Calculé | 63,38 | 6,20 | 12,32 | 12,47 |
| Trouvé | 63,55 | 6,17 | 12,39 | 12,56 |

### EXEMPLE 62 : 1-[(2R)-3,3-Diphénylalanyl]-N-[(6-amino-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3,3-diphénylalanine et du composé décrit dans la préparation D.

| Microanalyse élémentaire | | | | |
|---|---|---|---|---|
| | *%C* | *%H* | *%N* | *%Cl* |
| Calculé | 61,99 | 6,13 | 12,91 | 13,07 |
| Trouvé | 62,37 | 6,18 | 12,99 | 12,82 |

### EXEMPLE 63 : 1-[(2R)-3,3-Dicyclohexylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-3,3-dicyclohexylalanine et du composé décrit dans la préparation E.

| Microanalyse élémentaire | | | | |
|---|---|---|---|---|
| | *%C* | *%H* | *%N* | *%Cl* |
| Calculé | 61,26 | 8,33 | 12,32 | 12,47 |
| Trouvé | 61,03 | 8,21 | 12,48 | 12,77 |

### EXEMPLE 64 : 1-[(2R)-3,3-Dicyclohexylalanyl]-N-[6-amino-3-pyridyl)-méthyl]-(2S,3R)-2,3-méthano-homoprolinamide, dichlorhydrate

Le produit attendu est obtenu selon le procédé décrit dans l'exemple 2, à partir du composé décrit dans la préparation B, de *N*-tert-butyloxycarbonyl-(2*R*)-3,3-dicyclohexylalanine et du composé décrit dans la préparation D.

| Microanalyse élémentaire | | | | |
|---|---|---|---|---|
| | *%C* | *%H* | *%N* | *%Cl* |
| Calculé | 60,64 | 8,18 | 12,63 | 12,78 |
| Trouvé | 60,88 | 8,10 | 12,42 | 12,86 |

### EXEMPLE 65 : 1-[(2R)-Cyclohexylglycyl]-N-(4-amidinobenzyl)-(2S,3R)-2,3-méthano-homoprolinamide, dichlorhydrate

Le produit attendu est obtenu selon le procédé décrit dans l'exemple 2, à partir du composé décrit dans la préparation B, de *N*-tert-butyloxycarbonyl-(2*R*)-cyclohexylglycine et du composé décrit dans la préparation C.

### EXEMPLE 66 : 1-[N-Carboxyméthyl-(2R)-cyclohexylglycyl]-N-(4-amidinobenzyl)-(2S,3R)-2,3-méthano-homoprolinamide, chlorhydrate

Le produit attendu est obtenu selon le procédé décrit dans l'exemple 3, à partir du composé décrit dans l'exemple 65.

| Microanalyse élémentaire | | | | |
|---|---|---|---|---|
| | *%C* | *%H* | *%N* | *%Cl* |
| Calculé | 59,34 | 7,17 | 13,84 | 7,01 |
| Trouvé | 59,92 | 7,26 | 13,88 | 7,63 |

### EXEMPLE 67 : 1-[(2R)-Cyclohexylglycyl]-N-[(6-amino-2-méthyl-3-pyridyl)-méthyl] -(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-cyclohexylglycine et du composé décrit dans la préparation E.

| Microanalyse élémentaire | | | | |
|---|---|---|---|---|
| | *%C* | *%H* | *%N* | *%Cl* |
| Calculé | 55,93 | 7,47 | 14,82 | 15,01 |
| Trouvé | 56,51 | 7,52 | 14,83 | 15,20 |

### EXEMPLE 68 : 1-[(2R)-Cyclohexylglycyl]-N-[(6-amino-3-pyridyl)-méthyl]-(2S,3R)-2,3-méthano-homoprolinamide, dichlorhydrate

Le produit attendu est obtenu selon le procédé décrit dans l'exemple 1 à partir du composé décrit dans la préparation B, de *N*-tert-butyloxycarbonyl-(2*R*)-cyclohexylglycine et du composé décrit dans la préparation D.

| Microanalyse élémentaire | | | | |
|---|---|---|---|---|
| | *%C* | *%H* | *%N* | *%Cl* |
| Calculé | 55,02 | 7,26 | 15,28 | 15,47 |
| Trouvé | 55,50 | 7,36 | 15,41 | 15,91 |

### EXEMPLE 69 : 1-[(2R,3α)-3-Cyclohexyl-3-trifluorométhylalanyl]-N-(4-amidino-benzyl)-(2S,3R)-2,3-méthano-homoprolinamide, dichlorhydrate

### Stade A : 3-Cyclohexyl-4,4,4-trifluoro-2-buténoate de benzyle

A 10 mmoles d'hydrure de sodium en suspension dans 10 ml de diméthoxyéthane, sont ajoutées, à 0°C et au goutte à goutte, 10 mmoles de triméthoxyphosphonacétate de benzyle. Après 1 heure d'agitation à température ambiante sont ajoutées 10 mmoles de cyclohexyltrifluorométhylcétone. Le milieu réactionnel est agité 1 heure à température ambiante, puis versé dans 20 ml d'eau. La phase aqueuse est extraite à l'éther. La phase organique est lavée, séchée puis évaporée. On obtient une huile, qu'on distille sous vide à 100-110°C.

### Stade B : Acide (±)-3-cyclohexyl-3-trifluorométhyl-1-propionique

Le produit attendu est obtenu selon le procédé décrit dans le stade B de l'exemple 1 à partir du composé obtenu dans le stade précédent.

### Stade C : Chlorure de l'acide (+)-3-cyclohexyl-3-trifluorométhyl-1-propionique

A 10 mmoles de l'acide obtenu au stade précédent en solution dans du dichlorométhane à 0°C sont ajoutés 10 mmoles de chlorure de thionyle. Après retour à température ambiante et une nuit d'agitation, la solution est évaporée pour conduire au produit attendu.

### Stade D : (4R)-3-[3-Cyclohexyl-4,4,4-trifluoro-1-oxobutyl]-4-benzyl-2-oxazolidinone

A une solution de (4*R*)-(4-benzyl)-2-oxazolidinone (10 mmoles) dans le tétrahydrofurane est ajoutée une solution de n-butyllithium 2,5 M dans l'hexane (4,2 ml). Après 1 heure d'agitation à -78°C, on ajoute une solution du composé obtenu dans le stade précédent (10 mmoles) dans le tétrahydrofurane. Le mélange réactionnel est porté à 0°C puis coulé dans une solution aqueuse de chlorure d'ammonium. La phase aqueuse est extraite par l'acétate d'éthyle puis les phases organiques rassemblées sont lavées, séchées puis évaporées pour conduire au produit attendu sous forme d'un mélange de deux diastéréoisomères.

### Stade E : (4R)-3-[(3α)-3-Cyclohexyl-4,4,4-trifluoro-1-oxobutyl]-4-benzyl-2-oxazolidinone

Après séparation des diastéréoisomères du mélange obtenu dans le stade précédent par chromatographie sur gel de silice en utilisant comme éluant un mélange n-heptane/toluène (40/60), on obtient le produit attendu par évaporation de la première des 2 fractions dans l'ordre d'élution.

### Stade F : (4R)-3-[(2R,3α)-2-Azido-4,4,4-trifluoro-1-oxobutyl]-4-benzyl-2-oxazolidinone

A une solution à -78°C du composé décrit dans le stade précédent (10 mmoles) dans le tétrahydrofurane sont ajoutées au goutte à goutte une solution d'hexaméthyldisilazidure de potassium 0,5 M dans le toluène (10,5 mmoles), puis, après 1 heure d'agitation, une solution de trityl azide (11 mmoles) dans le tétrahydrofurane. Après 5 minutes d'agitation, de l'acide acétique glacial (3,5 ml) est ajouté, puis le mélange réactionnel est agité à température ambiante pendant 6 heures. Une solution de chlorure d'ammonium est ajoutée, puis, après extraction, la phase organique est lavée, séchée et évaporée pour conduire au produit attendu.

### Stade G : Acide (2R,3α)-2-arido-3-cyclohexyl-3-trifluorométhyl-1-propionique

A une solution à 0°C du composé obtenu dans le stade précédent (10 mmoles) dans le dioxane sont additionnées une solution aqueuse d'hydroxyde de lithium (12 mmoles) et une solution d'eau oxygénée à 30 % (5,7 ml). Après ½ heure d'agitation à 0°C, de l'acétate d'éthyle est ajouté, la phase organique est lavée, séchée puis évaporée pour conduire au produit attendu.

### Stade H : (2R,3a)-3-Cyclohexyl-3-trifluorométhylalanine

Le produit attendu est obtenu selon le procédé décrit dans le stade B de l'exemple 1 à partir du composé obtenu dans le stade précédent.

### Stade I : N-Tert-butyloxycarbonyl-(2R,3α)-3-cyclohexyl-3-trifluorométhylalanine

A 10 mmoles du composé décrit dans le stade précédent en solution dans le *tert*-butanol est ajoutée une solution de soude 1 N (11 mmoles) et du dicarbonate de di-*tert*-butyle (11 mmoles). Après 1 heure d'agitation, les solvants sont évaporés, le résidu est repris par de l'acétate d'éthyle, la phase organique est lavée, séchée et évaporée pour conduire au produit attendu.

### Stade J : 1-[N-Tert-butyloxycarbonyl-(2R,3α)-3-cyclohexyl-3-trifluorométhyl-alanyl]-(2S,3R)-2,3-méthano-homoproline

Le produit attendu est obtenu selon le procédé décrit dans les stades A et B de l'exemple 1 à partir du composé décrit dans le stade précédent et du composé décrit dans la préparation B.

### Stade K : 1-[(2R,3α)-3-Cyclohexyl-3-trifluorométhylalanyl]-N-(4-amidino-benzyl)-(2S,3R)-2,3-méthano-homoprolinamide, dichlorhydrate

Le produit attendu est obtenu selon le procédé décrit dans l'exemple 2, à partir du composé obtenu dans le stade précédent et du composé décrit dans la préparation C.

### EXEMPLE 70 : 1-[(2R,3β)-3-Cyclohexyl-3-trifluorométhylalanyl]-N-(4-amidino-benzyl)-(2S,3R)-2,3-méthano-homoprolinamide, dichlorhydrate

### Stade A : (4R)-3-[(3β)-3-Cyclohexyl-4,4,4-trifluoro-1-oxobutyl]-4-benzyl-2-oxazolidinone

Après séparation des diastéréoisomères du mélange décrit dans le stade D de l'exemple 69 par chromatographie sur gel de silice en utilisant comme éluant un mélange n-heptane/toluène (40/60), on obtient le produit attendu par évaporation de la deuxième des 2 fractions dans l'ordre d'élution.

### Stade B : 1-[N-Tert-butyloxycarbonyl-(2R,3β)-3-cyclohexyl-3-trifluorométhyl-alanyl]-(2S,3R)-2,3-méthano-homoproline

Le produit attendu est obtenu selon le procédé décrit dans les stades F à J de l'exemple 69, à partir du composé décrit dans le stade précédent.

### Stade C : 1-[(2R,3β)-3-Cyclohexyl-3-trifluorométhylalanyl]-N-(4-amidino-benzyl)-(2S,3R)-2,3-méthano-homoprolinamide, dichlorhydrate

Le produit attendu est obtenu selon le procédé décrit dans l'exemple 2, à partir du composé obtenu dans le stade précédent et du composé décrit dans la préparation C.

### EXEMPLE 71 : 1-[N-Carboxyméthyl-(2R,3α)-3-Cyclohexyl-3-trifluorométhylalanyl]-N-(4-amidinobenzyl)-(2S,3R)-2,3-méthano-homoprolinamide, chlorhydrate

Le produit attendu est obtenu selon le procédé décrit dans l'exemple 3, à partir du composé décrit dans l'exemple 69.

| Microanalyse élémentaire | | | | |
|---|---|---|---|---|
| | *%C* | *%H* | *%N* | *%Cl* |
| Calculé | 55,15 | 6,34 | 11,91 | 6,03 |
| Trouvé | 55,67 | 6,40 | 11,45 | 6,72 |

### EXEMPLE 72 : 1-[N-Carboxyméthyl-(2R,3β)-3-Cyclohexyl-3-trifluorométhylalanyl]-N-(4-amidinobenzyl)-(2S,3R)-2,3-méthano-homoprolinamide, chlorhydrate

Le produit attendu est obtenu selon le procédé décrit dans l'exemple 3, à partir du composé décrit dans l'exemple 70.

| Microanalyse élémentaire | | | | |
|---|---|---|---|---|
| | *%C* | *%H* | *%N* | *%Cl* |
| Calculé | 55,15 | 6,34 | 11,91 | 6,03 |
| Trouvé | 55,07 | 6,20 | 11,80 | 6,31 |

### EXEMPLE 73 : 1-[(2R,3α)-3-Cyclohexyl-3-trifluorométhylalanyl]-N-[(6-amino-2-méthyl-3-pyridyl)-méthyl]-(2S,3R)-23-méthano-homoprolinamide, dichlorhydrate

Le produit attendu est obtenu selon le procédé décrit dans l'exemple 1, à partir du composé décrit dans le stade J de l'exemple 69 et du composé décrit dans la préparation E.

### EXEMPLE 74 : 1-[(2R,3β)-3-Cyclohexyl-3-trifluorométhylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans le stade B de l'exemple 70 et du composé décrit dans la préparation E.

### EXEMPLE 75 : 1-[N-Carboxyméthyl-(2R,3α)-3-cyclohexyl-3-trifluorométhylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 3, à partir du composé décrit dans l'exemple 73.

### EXEMPLE 76 : 1-[N-Carboxyméthyl-(2R,3β)-3-cyclohexyl-3-trifluorométhylalanyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 3, à partir du composé décrit dans l'exemple 74.

### EXEMPLE 77 : 1-[N-Carboxyméthyl-(2R)-leucyl]-N-(4-amidinobenzyl)-(2S,3R)-2,3-méthano-homoprolinamide, chlorhydrate

### Stade A : 1-[(2R)-Leucyl]-N-(4-amidinobenzyl)-(2S,3R)-2,3-méthano-homoprolinamide, dichlorhydrate

Le produit attendu est obtenu selon le procédé décrit dans l'exemple 2, à partir du composé décrit dans la préparation B, de *N*-tert-butyloxycarbonyl-(2*R*)-leucine et du composé décrit dans la préparation C.

### Stade B : 1-[N-Carboxyméthyl-(2R)-leucyl]-N-(4-amidinobenzyl)-(2S,3R)-2,3-méthano-homoprolinamide, chlorhydrate

Le produit attendu est obtenu selon le procédé décrit dans l'exemple 3, à partir du composé obtenu dans le stade précédent

| Microanalyse élémentaire | | | | |
|---|---|---|---|---|
| | *%C* | *%H* | *%N* | *%Cl* |
| Calculé | 57,55 | 7,14 | 14,59 | 7,39 |
| Trouvé | 57,54 | 7,10 | 14,33 | 8,04 |

### EXEMPLE 78 : 1-[N-Méthyl-(2R)-phénylglycyl]-N-(6-amino-2-méthyl-3-pyridyl)-méthyl]-(2S,3R)-2,3-méthano-homoprolinamide, dichlorhydrate

### Stade A : 1-[(2R)-Phénylglycyl]-N-[(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 1, à partir du composé décrit dans la préparation B, de *N*-tert-butyloxycarbonyl-(2*R*)-phénylglycine et du composé décrit dans la préparation E.

### Stade B : 1-[N-Méthyl-(2R)-phénylglycyl]-N-(6-amino-2-méthyl-3-pyridyl)-méthyl]-(2S,3R)-2,3-méthano-homoprolinamide, dichlorhydrate

Le produit attendu est obtenu selon le procédé décrit dans les stades B et C de l'exemple 4 à partir du composé décrit dans le stade précédent.

### EXEMPLE 79 : 1-[N-Benzyl-(2R)-phénylglycyl]-N-(6-amino-2-méthyl-3-pyridyl)-méthyl]-(2S,3R)-2,3-méthano-homoprolinamide, dichlorhydrate

Le produit attendu est obtenu selon le procédé décrit dans l'exemple 5 à partir du composé décrit dans le stade A de l'exemple 78.

### EXEMPLE 80 : 1-[(2R)-3-Cyclohexyl-2-hydroxy-propionyl]-N-[(6-amino-2-méthyl-3-pyridyl)-méthyl]-(2S,3R)-2,3-méthano-homoprolinamide, chlorhydrate

### Stade A : 1-[(2R)-2-Acétoxy-3-cyclohexyl-propionyl]-N-[(6-amino-2-méthyl-3-pyridyl)-méthyl]- (2S,3R)-2,3-méthane-homoprolinamide, dichlorhydrate

Le produit attendu est obtenu selon le procédé décrit dans l'exemple 1 à partir du composé décrit dans le stade B de l'exemple 22, du composé décrit dans la préparation B et du composé décrit dans la préparation E.

### Stade B : 1-[(2R)-3-Cyclohexyl-2-hydroxy-propionyl]-N-[(6-amino-2-méthyl-3-pyridyl)-méthyl]-(2S,3R)-2,3-méthano-homoprolinamide, chlorhydrate

Le produit attendu est obtenu selon le procédé décrit dans les stades D et E de l'exemple 22, à partir du composé décrit dans le stade précédent.

| Microanalyse élémentaire | | | | |
|---|---|---|---|---|
| | *%C* | *%H* | *%N* | *%Cl* |
| Calculé | 61,25 | 7,82 | 12,42 | 7,86 |
| Trouvé | 61,66 | 7,76 | 12,29 | 8,43 |

### ETUDE PHARMACOLOGIQUE DES DERIVES DE L'INVENTION

### EXEMPLE 81 : Activité anti-coagulante, mesure de temps de thrombine et temps de céphaline activée chez l'homme

Afin d'évaluer l'activité anti-coagulante des composés de l'invention, le temps de thrombine (TT) et le temps de céphaline activée (TCA) ont été déterminés dans des échantillons de plasma humain. Un coagulomètre ST₄ (Diagnostica Stago, France) a été utilisé. Un plasma, pauvre en plaquettes, lyophylisé (Stago), est repris dans de l'eau distillée. Le TT est réalisé avec le réactif Thrombine Prest et le TCA avec le réactif Céphaline PTT Automate.

L'inhibiteur ou le solvant (10 µl) est additionné au plasma (90 µl), puis incubé 2 minutes à 37°C. 100 µl de Thrombine Prest (TT) ou de Céphaline PTT Automate (TCA) sont ajoutés en déclenchant le chronomètre.

Dans ces conditions, le TT est de l'ordre de 18 secondes, le TCA est de l'ordre de 12 secondes. L'activité d'un antagoniste est évaluée par sa capacité de prolonger le TT et le TCA par rapport au témoin. L'effet des inhibiteurs est exprimé par la concentration en µM qui multiplie de 2 le temps de coagulation (Ctt₂).

Les composés de l'invention ont produit des prolongations des temps de coagulation très importantes et les Ctt₂ sont exemplifiés dans le tableau 1 ci-dessous :

**Tableau 1 :**

| Exemple | TT C_{tt₂}(µM) | TCA C_{tt₂}(µM) |
|---|---|---|
| 2 | 0,07 | 0,52 |
| 3 | 0,06 | 0,56 |
| 9 | 0,20 | 0,86 |
| 11 | 0,06 | 0,59 |
| 13 | 0,18 | 2,30 |
| 14 | 0,19 | 2,20 |
| 16 | 0,09 | 0,42 |
| 17 | 0,11 | 0,86 |
| 24 | 0,03 | 0,58 |
| 25 | 0,08 | 0,45 |
| 28 | 0,31 | 2,30 |
| 29 | 0,46 | 2,80 |
| 31 | 0,17 | 1,30 |
| 32 | 0,12 | 1,09 |
| 33 | 0,11 | 1,10 |
| 61 | 0,05 | 0,47 |

### EXEMPLE 82 : Inhibition de la thrombine et de protéases à sérine de la fibrinolyse

Pour évaluer in vitro l'activité inhibitrice des produits de l'invention sur la thrombine humaine (Sigma, activité spécifique 3230 UNIH/mg), le fibrinogène humain purifié (4 mM, Stago) (Fg) a été ajouté à une quantité donnée de thrombine (0,7 nM) préalablement incubée avec ou sans l'inhibiteur à tester (20°C, 10 minutes).

Pour évaluer in vitro la sélectivité de ces produits vis-à-vis de la plasmine, le même protocole a été appliqué à la plasmine humaine purifiée (2 nM, Stago), en utilisant pour substrat un peptide paranitroanilidé : <Glu-Phe-Lys-pNA (0,50 mM, S 2403, Kabi).

Inhibiteurs, enzymes et substrats sont dilués dans le même tampon (tampon phosphate 0,01 mM, pH 7,4, contenant 0,12 M de chlorure de sodium et 0,05 % de sérum albumine bovine) puis distribués dans une microplaque en polystyrène sous un volume de 50 µl. La fibrine formée par la thrombine ou par le paranitroanilide libéré par l'action de la protéase à sérine est mesurée spectrophotométriquement à 405 nm après 15 à 30 minutes de réaction à 20°C.

Le tableau 2 ci-dessous donne la concentration des composés en nM inhibant 50 % de l'activité enzymatique (CI₅₀) de la thrombine par rapport au contrôle sans produit. Les résultats obtenus démontrent que les composés de l'invention sont des inhibiteurs puissants de la thrombine humaine vis-à-vis du fibrinogène humain.

Le tableau 3 ci-dessous donne la concentration des composés en nM inhibant 50 % de l'activité enzymatique (CI₅₀) des protéases de la fibrinolyse. Les résultats obtenus démontrent que les composés possèdent une sélectivité très importante vis-à-vis des protéases à sérine de la fibrinolyse.

**Tableau 2 :**

| Exemple | CI₅₀ (nM) |
|---|---|
| 2 | 7,9 |
| 3 | 5,3 |
| 9 | 6,0 |
| 11 | 4,0 |
| 13 | 1,2 |
| 14 | 1,0 |
| 16 | 1,0 |
| 17 | 28,0 |
| 24 | 2,3 |
| 25 | 2,0 |
| 28 | 26,0 |
| 29 | 44,0 |
| 31 | 5,1 |
| 32 | 7,1 |
| 33 | 5,1 |
| 61 | 1,0 |
| 63 | 8,8 |

**Tableau 3 :**

| Exemple | CI₅₀ (nM) | | |
|---|---|---|---|
| | Plasmine | t.PA | v.PA |
| 2 | > 33.000 | 33.000 | > 33.000 |
| 11 | 7.200 | > 33.000 | 2.800 |
| 24 | 4.700 | 2.800 | > 33.000 |
| 28 | > 33.000 | > 33.000 | > 33.000 |
| 29 | > 33.000 | > 33.000 | > 33.000 |
| 31 | > 33.000 | > 33.000 | > 33.000 |
| 33 | > 33.000 | > 33.000 | > 33.000 |
| 61 | > 33.000 | 33.000 | > 33.000 |
| 63 | > 33.000 | > 33.000 | > 33.000 |

### EXEMPLE 83 : Activité anti-coagulante après administration per os chez le chien

Des chiens mâles ou femelles de 11-28 kg sont traités par voie orale avec les produits de l'invention (5 ou 10 mg/kg). Les temps de coagulation (TT, TCA) sont déterminés dans des échantillons de plasma des chiens 10 min avant et 30 min, 1 heure, 2 heures, 4 heures et 6 heures après l'administration des produits. Les mesures des temps de coagulation sont effectuées comme décrits dans l'exemple 81.

Dans les conditions de nos expériences, le TT est de l'ordre de 19 secondes et le TCA de l'ordre de 18 secondes.

Les substances de l'invention augmentent de façon importante les TT et les TCA chez les animaux. Le tableau 4 résume les résultats obtenus. Les résultats montrent l'augmentation maximale des TT et TCA obtenue après traitement p.o. des chiens. Les valeurs montrent le nombre de fois que le temps initial est augmenté.

**Tableau 4**

| *Augmentations de TT et TCA (nombre de fois le temps initial)* | | | |
|---|---|---|---|
| Exemple | dose (mg/kg) | TT | TCA |
| 2 | 5 | 3,8 | 1,4 |
| 3 | 5 | 8,4 | 2,1 |
| 11 | 5 | 5,6 | 2,3 |
| 17 | 5 | 16,0 | 3,7 |
| 24 | 5 | 6,7 | 1,2 |
| 28 | 10 | 6,2 | 1,8 |
| 29 | 10 | 6,2 | 1,7 |
| 33 | 5 | 7,1 | 1,5 |
| 61 | 5 | 14,2 | 2,0 |

### EXEMPLE 84 : Composition pharmaceutique

| Formule de préparation pour 1000 comprimés dosés à 10 mg : | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I): 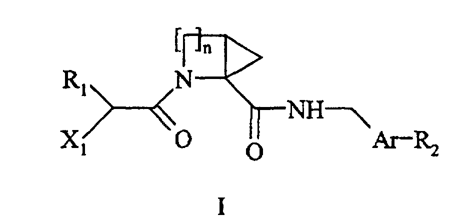 dans laquelle :
◆ n représente 2 ou 3,
◆ R₁ représente un groupement cycloalkyle (C₃-C₈), un groupement phényle éventuellement substitué ou un groupement alkyle (C₁-C₆) linéaire ou ramifié éventuellement substitué par un ou plusieurs groupements, identiques ou différents, halogène, cycloalkyle (C₃-C₈) ou phényle éventuellement substitué,
◆ R₂ représente :
- un groupement amino,
- un groupement amidino substitué éventuellement par un ou plusieurs groupements identiques ou différents, alkyle (C₁-C₆) linéaire ou ramifié, ou hydroxy,
- un groupement guanidino substitué éventuellement par un groupement alkyle (C₁-C₆) linéaire ou ramifié,
- ou un groupement isothiouréïdo substitué éventuellement par un groupement alkyle (C₁-C₆) linéaire ou ramifié,
◆ Ar représente un groupement aryle ou un groupement hétéroaryle azoté monocyclique,
◆ X₁ représente un groupement hydroxy, un groupement amino ou un groupement -NHR₃,
• R₃ représente un groupement propargyle, un groupement iminométhyle, un groupement alkylsulfonyle (C₁-C₆) linéaire ou ramifié, un groupement arylalkylsulfonyle (C₁-C₆) linéaire ou ramifié, un groupement -CONR'₃R"₃ ou un groupement alkyle (C₁-C₆) linéaire ou ramifié éventuellement substitué par :
- un groupement -CO₂R'_{3,}
- un groupement -CONR'₃R"₃,
- un groupement hétérocyclique,
- un groupement aminosulfonyle,
- un groupement aryle,
- ou un groupement hétéroaryle,
* R'₃ et R"₃, identiques ou différents, représentent chacun un atome d'hydrogène, ou un groupement alkylsulfonyle (C₁-C₆) linéaire ou ramifié, un groupement aryle, un groupement alkyle (C₁-C₆) linéaire ou ramifié (éventuellement substitué par un groupement carboxy, alkoxycarbonyle (C₁-C₆) linéaire ou ramifié ou carbamoyle), ou forment avec l'atome d'azote qui les portent un groupement hétérocyclique,
leurs isomères ainsi que leurs sels d'addition à un acide ou à une base pharmaceutiquement acceptable,
étant entendu que par groupement phényle éventuellement substitué, on entend substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié et amino (substitué éventuellement par un ou plusieurs groupements alkyle (C₁-C₆) linéaire ou ramifié),
par groupement hétérocyclique, on entend un groupement mono- ou bicyclique, saturé ou insaturé, de 5 à 12 chaînons contenant un, deux ou trois hétéroatomes choisis parmi oxygène, azote ou soufre, étant entendu que l'hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, oxo, hydroxy, trihalogénoalkyle (C₁-C₆) linéaire ou ramifié et amino (substitué éventuellement par un ou plusieurs alkyle (C₁-C₆) linéaire ou ramifié),
par groupement aryle, on entend phényle, biphénylyle, naphtyle, chacun de ces groupements étant éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs groupements alkyle (C₁-C₆) linéaire ou ramifié) et carboxy,
et par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié).

2. Composé de formule (I) selon la revendication 1 tel que R₁ représente un groupement cycloalkyle (C₃-C₈) ou un groupement alkyle (C₁-C₆) linéaire ou ramifié éventuellement substitué.

3. Composé de formule (I) selon la revendication 1 tel que R₂ représente un groupement amino ou amidino.

4. Composé de formule (I) selon la revendication 1 tel que Ar représente un groupement phényle éventuellement substitué ou un groupement pyridyle éventuellement substitué.

5. Composé de formule (I) selon la revendication 1 tel que Xi représente un groupement amino ou un groupement -NHR₃ dans lequel R₃ représente un groupement arylalkylsulfonyle (C₁-C₆) linéaire ou ramifié, ou un groupement alkyle (C₁-C₆) linéaire ou ramifié éventuellement substitué.

6. Composé de formule (I) selon la revendication 1 qui est le 1-[(2*R*)-3,3-diphénylalanyl]-*N*-(4-amidinobenzyl)-(2*S*,3*R*)-2,3-méthano-prolinamide, ses isomères ainsi que ses sels d'addition à un acide pharmaceutiquement acceptable.

7. Composé de formule (I) selon la revendication 1 qui est le 1-[*N*-carboxyméthyl-(2*R*)-cyclohexylglycyl]-*N*-(4-amidinobenzyl)-(2*S*,3*R*)-2,3-méthano-prolinamide, ses isomères ainsi que ses sels d'addition à un acide pharmaceutiquement acceptable.

8. Composé de formule (I) selon la revendication 1 qui est le 1-[(2*R*)-3-cyclohexylalanyl]-*N*-[(6-amino-2-méthyl-3-pyridyl)-méthyl]-(2*S*,3*R*)-2,3-méthano-homoprolinamide, ses isomères ainsi que ses sels d'addition à un acide pharmaceutiquement acceptable.

9. Composé de formule (I) selon la revendication 1 qui est le 1-[*N*-méthyl-(2*R*)-3-cyclohexylalanyl]-*N*-[(6-amino-2-méthyl-3-pyridyl)-méthyl]-(2*S*,3*R*)-2,3-méthano-homoprolinamide, ses isomères ainsi que ses sels d'addition à un acide pharmaceutiquement acceptable.

10. Composé de formule (I) selon la revendication 1 qui est le 1-[(2R)-3,3-diphénylalanyl]-*N*-(4-amidinobenzyl)-(2*S*,3*R*)-2,3-méthano-homoprolinamide, ses isomères ainsi que ses sels d'addition à un acide pharmaceutiquement acceptable.

11. Composé de formule (I) selon la revendication 1 qui est le 1-[(2*R*)-3,3-dicyclohexylalanyl]-*N*-[(6-amino-2-méthyl-3-pyridyl)-méthyl]-(2*S*,3*R*)-2,3-méthano-homoprolinamide, ses isomères ainsi que ses sels d'addition à un acide pharmaceutiquement acceptable.

12. Procédé de préparation des composés de formule (I) selon la revendication 1
**caractérisé en ce que** l'on met en réaction un composé de formule (II) : 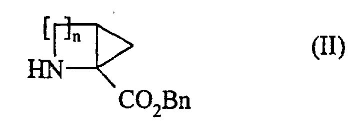 dans laquelle n a la même signification que dans la formule (I), et Bn représente le groupement benzyle,
avec un composé de formule (III) : 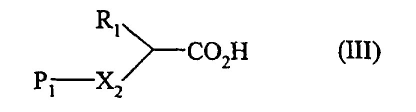 dans laquelle R₁ a la même signification que dans la formule (I), X₂ représente un atome d'oxygène ou -NH-, et P₁ représente un groupement protecteur de la fonction hydroxy ou amino,
en présence d'un agent de couplage peptidique,
pour conduire au composé de formule (IV) : 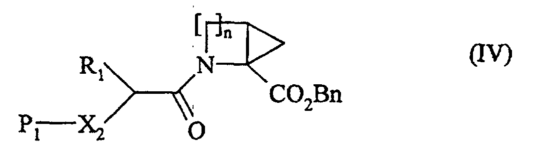 dans laquelle n, R₁, X₂, Bn et P₁ ont la même signification que précédemment,
composé de formule (IV) que l'on transforme ensuite en composé de formule (V) : 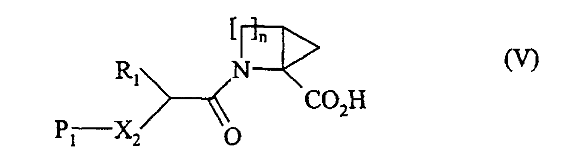 dans laquelle n, R₁, X₂ et P₁ ont la même signification que précédemment, par hydrogénation catalytique ou par saponification,
composé de formule (V) que l'on met ensuite en réaction avec un composé de formule (VI) : 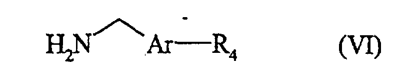 dans laquelle Ar a la même signification que dans la formule (I), et R₄ représente R₂ ou R₂-P₂, R₂ ayant la même signification que dans la formule (I), et P₂ représentant un groupement protecteur de la fonction amino,
en présence d'un agent de couplage peptidique,
pour conduire au composé de formule (VII) : 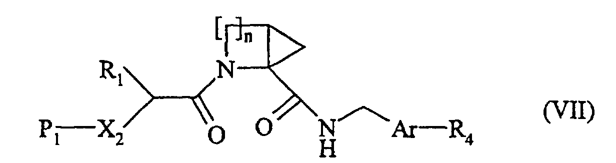 dans laquelle n, R₁, R₄, Ar, X₂ et P₁ ont la même signification que précédemment,
composé de formule (VII) que l'on transforme ensuite en composé de formule (I) par une ou plusieurs réactions de déprotection, d'alkylation ou d'amination réductrice,
composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

13. Composition pharmaceutique contenant comme principe actif un composé selon l'une quelconque des revendications 1 à 11, en combinaison avec un ou plusieurs véhicules inertes, non toxiques et pharmaceutiquement acceptables.

14. Composition pharmaceutique selon la revendication 13 utile en tant qu'inhibiteur de protéases à sérine apparentées à la trypsine.

15. Composition pharmaceutique selon la revendication 14 utile en tant qu'inhibiteur de thrombine.

## Patentansprüche

1. Verbindung der Formel (I): 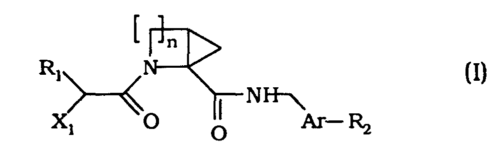 in der:
◆ n 2 oder 3 darstellt.
◆ R₁ eine (C₃-C₈)-Cycloalkylgruppe, eine gegebenenfalls substituierte Phenylgruppe oder eine gegebenenfalls durch ein oder mehrere gleichartige oder verschiedene Halogenatome, (C₃-C₈)-Cycloalkylgruppen oder gegebenenfalls substituierte Phenylgruppen substituierte (C₁-C₆)-Alkylgruppe bedeutet,
◆ R₂:
- eine Aminogruppe.
- eine gegebenenfalls durch eine oder mehrere gleichartige oder verschiedene, geradkettige oder verzweigte (C₁-C₆)-Alkylgruppen oder Hydroxygruppen substituierte Amidinogruppe,
- eine gegebenenfalls durch eine geradkettige oder verzweigte (C₁-C₆)-Alkylgruppe substituierte Guanidinogruppe,
- oder eine gegebenenfalls durch eine geradkettige oder verzweigte (C₁-C₆)-Alkylgruppe substituierte Isothioureidogruppe darstellt,
◆ Ar eine Arylgruppe oder eine monocyclische stickstoffhaltige Heteroarylgruppe bedeutet,
◆ X₁ eine Hydroxygruppe, eine Aminogruppe oder eine Gruppe -NHR₃ darstellt,
worin
• R₃ eine Propargylgruppe, eine Iminomethylgruppe, eine geradkettige oder verzweigte (C₁-C₆)-Alkylsulfonylgruppe, eine geradkettige oder verzweigte (C₁-C₆)-Arylalkylsulfonylgruppe, eine Gruppe -CONR'₃R"₃ oder eine geradkettige oder verzweigte (C₁-C₆)-Alkylgruppe bedeutet, die gegebenenfalls substituiert ist durch:
- eine Gruppe -CO₂R'₃.
- eine Gruppe -CONR'₃R"₃,
- eine heterocyclische Gruppe,
- eine Aminosulfonylgruppe,
- eine Arylgruppe.
- oder eine Heteroarylgruppe,
worin
* R'₃ und R"₃, die gleichartig oder verschieden sind, jeweils ein Wasserstoffatom oder eine geradkettige oder verzweigte (C₁-C₆)-Alkylsulfonylgruppe, eine Arylgruppe, eine geradkettige oder verzweigte (gegebenenfalls durch eine Carboxygruppe, eine geradkettige oder verzweigte (C₁-C₆)-Alkoxycarbonylgruppe oder eine Carbamoylgruppe substituierte) (C₁-C₆)-Alkylgruppe darstellen oder gemeinsam mit dem sie tragenden Stickstoffatom eine heterocyclische Gruppe bilden,
deren Isomeren sowie deren Additionssalze mit einer pharmazeutisch annehmbaren Säure oder Base,
wobei es sich versteht, daß unter einer gegebenenfalls substituierten Phenylgruppe eine Substitution durch eine oder mehrere, gleichartige oder verschiedene Gruppen ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl und (gegebenenfalls durch eine oder mehrere geradkettige oder verzweigte (C₁-C₆)-Alkylgruppen substituiertem) Amino zu verstehen ist,
unter einer heterocyclischen Gruppe eine mono- oder bicyclische, gesättigte oder ungesättigte Gruppe mit 5 bis 12 Kettengliedern, die ein, zwei oder drei Heteroatome ausgewählt aus Sauerstoff, Stickstoff oder Schwefel enthält, zu verstehen ist, wobei es sich versteht, daß der Heterocyclus gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Alkoxy, Oxo, Hydroxy, geradkettigem oder verzweigtem (C₁-C₆)-Trihalogenalkyl und (gegebenenfalls durch eine oder mehrere geradkettige oder verzweigte (C₁-C₆)-Alkylgruppen substituiertem) Amino substituiert sein kann,
unter einer Arylgruppe Phenyl, Biphenyl und Naphthyl zu verstehen ist, wobei jede dieser Gruppen gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl, (gegebenenfalls durch eine oder mehrere geradkettigen oder verzweigte (C₁-C₆)-Alkylgruppen substituiertem) Amino und Carboxy substituiert ist.
und unter einer Heteroarylgruppe eine aromatische mono- oder bicyclische Gruppe mit 5 bis 12 Kohlenstoffatomen, die ein, zwei oder drei Heteroatome ausgewählt aus Sauerstoff, Stickstoff oder Schwefel enthält, zu verstehen ist, wobei es sich versteht, daß die Heteroarylgruppe gegebenenfalls durch eine oder mehrere gleichartige oder verschiedene Gruppen ausgewählt aus Halogen, geradkettigem oder verzweigtem (C₁-C₆)-Alkyl, Hydroxy, geradkettigem oder verzweigtem (C₁-C₆)-Alkoxy, Trihalogenmethyl und (gegebenenfalls durch eine oder mehrere geradkettige oder verzweigte (C₁-C₆)-Alkylgruppen substituiertem) Amino substituiert sein kann.

2. Verbindung der Formel (I) nach Anspruch 1, worin R₁ eine (C₃-C₈)-Cycloalkylgruppe oder eine geradkettige oder verzweigte, gegebenenfalls substituierte (C₁-C₆)-Alkylgruppe bedeutet.

3. Verbindung der Formel (I) nach Anspruch 1, worin R₂ eine Amino- oder Amidinogruppe darstellt.

4. Verbindung der Formel (I) nach Anspruch 1, worin Ar eine gegebenenfalls substituierte Phenylgruppe oder eine gegebenenfalls substituierte Pyridylgruppe bedeutet.

5. Verbindung der Formel (I) nach Anspruch 1, worin X₁ eine Aminogruppe oder eine Gruppe -NHR₃ darstellt, worin R₃ eine geradkettige oder verzweigte (C₁-C₆)-Arylalkylsulfonylgruppe oder eine geradkettige oder verzweigte, gegebenenfalls substituierte (C₁-C₆)-Alkylgruppe bedeutet.

6. Verbindung der Formel (I) nach Anspruch 1, nämlich 1-[(2*R*)-3,3-Diphenylalanyl]-*N*-(4-amidinobenzyl)-(2*S*,3*R*)-2,3-methano-prolinamid, dessen Isomeren sowie dessen Additionssalze mit einer pharmazeutisch annehmbaren Säure.

7. Verbindung der Formel (I) nach Anspruch 1, nämlich 1-[*N*-Carboxymethyl-(2*R*)-cyclohexylglycyl]-*N*-(4-amidinobenzyl)-(2*S*,3*R*)-2,3-methano-prolinamid, dessen Isomeren sowie dessen Additionssalze mit einer pharmazeutisch annehmbaren Säure.

8. Verbindung der Formel (I) nach Anspruch 1, nämlich 1-[(2R)-3-Cyclohexylalanyl]-*N*-[(6-amino-2-methyl-3-pyridyl)-methyl]-(2*S*,3*R*)-2,3-methano-homoprolinamid, dessen Isomeren sowie dessen Additionssalze mit einer pharmazeutisch annehmbaren Säure.

9. Verbindung der Formel (I) nach Anspruch 1, nämlich 1-[*N*-Methyl-(2*R*)-3-cyclohexylalanyl]-*N*-[(6-amino-2-methyl-3-pyridyl)-methyl]-[2*S*,3*R*)-2,3-methano-homoprolinamid, dessen Isomeren sowie dessen Additionssalze mit einer pharmazeutisch annehmbaren Säure.

10. Verbindung der Formel (I) nach Anspruch 1, nämlich 1-[(2*R*)-3,3-Diphenylalanyl]-*N*-(4-amidinobenzyl)-(2*S*,3*R*)-2,3-methano-homoprolinamid, dessen Isomeren sowie dessen Additionssalze mit einer pharmazeutisch annehmbaren Säure.

11. Verbindung der Formel (I) nach Anspruch 1, nämlich 1-[(2R)-3,3-Dicyclohexylalanyl]-*N*-[(6-amino-2-methyl-3-pyridyl)-methyl]-(2*S*,3*R*)-2,3-methano-homoprolinamid, dessen Isomeren sowie dessen Additionssalze mit einer pharmazeutisch annehmbaren Säure.

12. Verfahren zur Herstellung der Verbindungen der Formel (I) nach Anspruch 1, **dadurch gekennzeichnet, daß** man eine Verbindung der Formel (II): 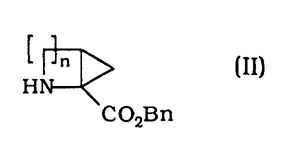 in der n die bezüglich der Formel (I) angegebenen Bedeutungen besitzt und Bn eine Benzylgruppe darstellt,
mit einer Verbindung der Formel (III): 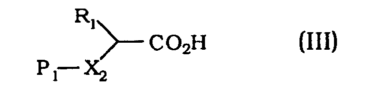 in der R₁ die bezüglich der Formel (1) angegebenen Bedeutungen besitzt, X₂ ein Sauerstoffatom oder -NH- und P₁ eine Schutzgruppe für die Hydroxy- oder Aminofunktion bedeuten,
in Gegenwart eines Peptid-Kupplungsmittels umsetzt,
zur Bildung der Verbindung der Formel (IV): 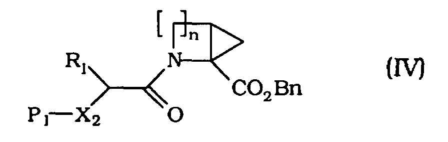 in der n, R₁, X₂, Bn und P₁ die oben angegebenen Bedeutungen besitzen,
welche Verbindung der Formel (IV) man anschließend durch katalytische Hydrierung oder durch Verseifung in die Verbindung der Formel (V) umwandelt: 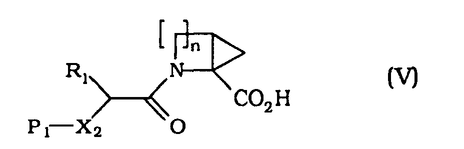 in der n, R₁, X₂ und P₁ die oben angegebenen Bedeutungen besitzen,
welche Verbindung der Formel (V) man anschließend mit einer Verbindung der Formel (VI): 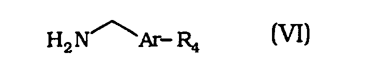 in der Ar die bezüglich der Formel (I) angegebenen Bedeutungen besitzt und R₄ R₂ oder R₂-P₂ darstellt, worin R₂ die bezüglich der Formle (I) angegebenen Bedeutungen besitzt und P₂ eine Schutzgruppe für die Aminofunktion bedeutet,
in Gegenwart eines Peptid-Kupplungsmittels umsetzt.
zur Bildung der Verbindung der Formel (VII): 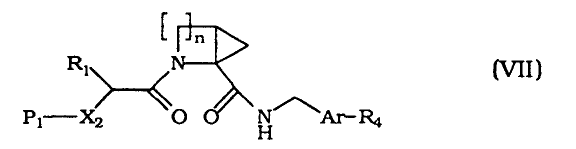 in der n, R₁, R₄, Ar, X₂ und P₁ die oben angegebenen Bedeutungen besitzen,
welche Verbindung der Formel (VII) man anschließend durch eine oder mehrere Reaktionen der Schutzgruppenabspaltung, der Alkylierung oder der reduktiven Aminierung in die Verbindung der Formel (I) umwandelt,
welche Verbindung der Formel (I) man gegebenenfalls mit Hilfe einer klassischen Reinigungsmethode reinigt, gewünschtenfalls mit Hilfe einer klassischen Trennungsmethode in die Isomeren auftrennt und gewünschtenfalls mit Hilfe einer pharmazeutisch annehmbaren Säure oder Base in die Additionssalze umwandelt.

13. Pharmazeutische Zubereitung enthaltend als Wirkstoff eine Verbindung nach einem der Ansprüche 1 bis 11 in Kombination mit einem oder mehreren inerten, nichttoxischen und pharmazeutisch annehmbaren Trägermaterialien.

14. Pharmazeutische Zubereitung nach Anspruch 13 geeignet als Inhibitor von mit Trypsin verwandten Serinproteasen.

15. Pharmazeutische Zubereitung nach Anspruch 14 geeignet als Thrombininhibitor.

## Claims

1. Compound of formula (I) : 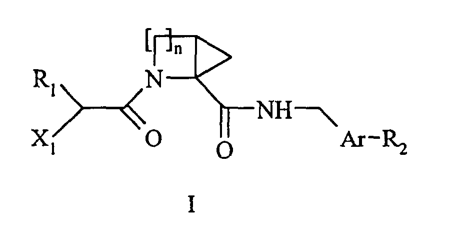 wherein :
◆ n represents 2 or 3.
◆ R₁ represents a (C₃-C₈)cycloalkyl group, an optionally substituted phenyl group or a linear or branched (C₁-C₆)alkyl group optionally substituted by one or more identical or different groups selected from halogen, (C₃-C₈)cycloalkyl and optionally substituted phenyl,
◆ R₂ represents :
- an amino group,
- an amidino group optionally substituted by one or more identical or different groups selected from linear or branched (C₁-C₆)alkyl and hydroxy,
- a guanidino group optionally substituted by a linear or branched (C₁-C₆)alkyl group, or
- an isothioureido group optionally substituted by a linear or branched (C₁-C₆)alkyl group,
◆ Ar represents an aryl group or a monocyclic nitrogen-containing heteroaryl group,
◆ X₁ represents a hydroxy group , an amino group or an -NHR₃ group,
• R₃ represents a propargyl group, an iminomethyl group, a linear or branched (C₁-C₆)alkylsulphonyl group, an aryl(C₁-C₆)alkylsulphonyl group in which the alkyl moiety may be linear or branched, a -CONR'₃R"₃ group or a linear or branched (C₁-C₆)alkyl group optionally substituted by :
- a -CO₂R'₃ group,
- a -CONR'₃R"₃ group,
- a heterocyclic group,
- an aminosulphonyl group,
- an aryl group, or
- a heteroaryl group,
* R'₃ and R"₃, which may be identical or different, each represents a hydrogen atom, or a linear or branched (C₁-C₆)alkylsulphonyl group, an aryl group, a linear or branched (C₁-C₆)alkyl group (optionally substituted by a carboxy group, a linear or branched (C₁-C₆)alkoxycarbonyl group or a carbamoyl group) or form, with the nitrogen atom carrying them, a heterocyclic group,
isomers thereof, and also addition salts thereof with a pharmaceutically acceptable acid or base,
wherein an optionally substituted phenyl group is to be understood as being substituted by one or more identical or different groups selected from halogen, linear or branched (C₁-C₆)alkyl, linear or branched (C₁-C₆)alkoxy, hydroxy, linear or branched trihalo-(C₁-C₆)alkyl, and amino (optionally substituted by one or more linear or branched (C₁-C₆)alkyl groups),
a heterocyclic group is to be understood as meaning a saturated or unsaturated, mono- or bi-cyclic group having from 5 to 12 ring members containing one, two or three hetero atoms selected from oxygen, nitrogen and sulphur, it being understood that the heterocycle may be optionally substituted by one or more identical or different groups selected from halogen, linear or branched (C₁-C₆)alkyl, linear or branched (C₁-C₆)alkoxy, oxo, hydroxy, linear or branched trihalo-(C₁-C₆)alkyl, and amino (optionally substituted by one or more linear or branched (C₁-C₆)alkyl groups),
an aryl group is to be understood as meaning phenyl, biphenylyl or naphthyl, each of those groups being optionally substituted by one or more identical or different groups selected from halogen, linear or branched (C₁-C₆)alkyl, linear or branched (C₁-C₆)alkoxy, hydroxy, linear or branched trihalo-(C₁-C₆)alkyl, amino (optionally substituted by one or more linear or branched (C₁-C₆)alkyl groups) and carboxy,
a heteroaryl group is to be understood as meaning an aromatic mono- or bi-cyclic group having from 5 to 12 ring members containing one, two or three hetero atoms selected from oxygen, nitrogen and sulphur, it being understood that heteroaryl may be optionally substituted by one or more identical or different groups selected from halogen, linear or branched (C₁-C₆)alkyl, hydroxy, linear or branched (C₁-C₆)-alkoxy, trihalomethyl, and amino (optionally substituted by one or more linear or branched (C₁-C₆)alkyl groups).

2. Compound of formula (I) according to claim 1, wherein R₁ represents a (C₃-C₈)-cycloalkyl group or an optionally substituted linear or branched (C₁-C₆)alkyl group.

3. Compound of formula (I) according to claim 1, wherein R₂ represents an amino or amidino group.

4. Compound of formula (I) according to claim 1, wherein Ar represents an optionally substituted phenyl group or an optionally substituted pyridyl group.

5. Compound of formula (I) according to claim 1, wherein X₁ represents an amino group or an -NHR₃ group wherein R₃ represents an aryl-(C₁-C₆)alkylsulphonyl group in which the alkyl moiety may be linear or branched, or an optionally substituted linear or branched (C₁-C₆)alkyl group

6. Compound of formula (I) according to claim 1, which is 1-[(2*R*)-3,3-diphenylalanyl]-*N*-(4-amidinobenzyl)-(2*S*,3*R*)-2,3-methano-prolinamide, its isomers, and also addition salts thereof with a pharmaceutically acceptable acid.

7. Compound of formula (I) according to claim 1, which is 1-[*N*-carboxymethyl-(2*R*)-cyclohexylglycyl]-*N*-(4-amidinobenzyl)-(2*S*,3*R*)-2,3-methano-prolinamide, its isomers, and also addition salts thereof with a pharmaceutically acceptable acid.

8. Compound of formula (I) according to claim 1, which is 1-[(2*R*)-3-cyclohexylalanyl]-*N*-[(6-amino-2-methyl-3-pyridyl)methyl]-(2*S*,3*R*)-2,3-methano-homoprolinamide, its isomers, and also addition salts thereof with a pharmaceutically acceptable acid.

9. Compound of formula (I) according to claim 1, which is 1-[*N*-methyl-(2*R*)-3-cyclo-hexylalanyl]-*N*-[(6-amino-2-methyl-3-pyridyl)methyl]-(2*S*,3*R*)-2,3-methano-homoprolinamide, its isomers, and also addition salts thereof with a pharmaceutically acceptable acid.

10. Compound of formula (I) according to claim 1, which is 1-[(2R)-3,3-diphenylalanyl]-*N*-(4-amidinobenzyl)-(2*S*,3*R*)-2,3-methano-homoprolinamide, its isomers, and also addition salts thereof with a pharmaceutically acceptable acid.

11. Compound of formula (I) according to claim 1, which is 1-[(2R)-3,3-dicyclohexylalanyl]-*N*-[(6-amino-2-methyl-3-pyridyl)methyl]-(2*S*,3*R*)-2,3-methano-homoprolinamide, its isomers, and also addition salts thereof with a pharmaceutically acceptable acid.

12. Process for the preparation of compounds of formula (I) according to claim 1, **characterized in that** a compound of formula (II) : 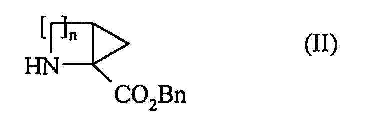 wherein n is as defined for formula (I) and Bn represents a benzyl group, is reacted
with a compound of formula (III) : 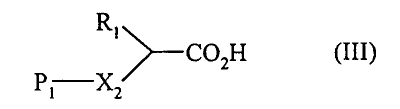 wherein R₁ is as defined for formula (I), X₂ represents an oxygen atom or -NH-, and P₁ represents an amino function-protecting or hydroxy function-protecting group, in the presence of a peptide coupling agent,
to yield a compound of formula (IV) : 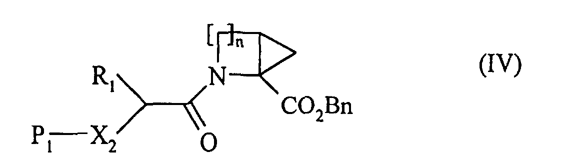 wherein n, R₁, X₂, Bn and P₁ are as defined hereinbefore,
which compound of formula (IV) is then converted, by catalytic hydrogenation or by hydrolysis, into a compound of formula (V): 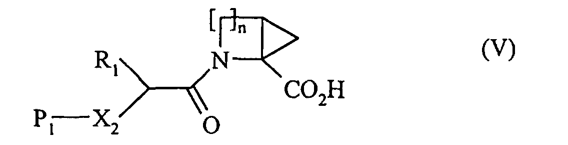 wherein n, R₁, X₂ and P₁ are as defined hereinbefore,
which compound of formula (V) is then subjected to reaction with a compound of formula (VI) : 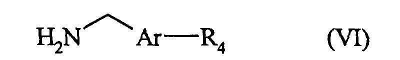 wherein Ar is as defined for formula (I) and R₄ represents R₂ or R₂-P₂, R₂ being as defined for formula (I) and P₂ representing an amino function-protecting group,
in the presence of a peptide coupling agent,
to yield a compound of formula (VII) : 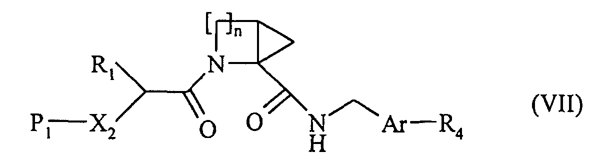 wherein n, R₁, R₄, Ar, X₂ and P, are as defined hereinbefore,
which compound of formula (VII) is then converted into a compound of formula (I) by one or more deprotection, alkylation or reductive amination reactions,
which compound of formula (I) is purified, if necessary, according to a conventional purification technique, is separated, if desired, into its isomers according to a conventional separation technique, and is converted, if desired, into an addition salt with a pharmaceutically acceptable acid or base.

13. Pharmaceutical composition comprising as active ingredient a compound according to any one of claims 1 to 11, in combination with one or more pharmaceutically acceptable, inert, non-toxic carriers.

14. Pharmaceutical composition according to claim 13 for use as a trypsin-related serine protease inhibitor.

15. Pharmaceutical composition according to claim 14 for use as a thrombin inhibitor.
